# EUROPEAN PATENT APPLICATION

(11) **EP 3 608 983 A1**
(43) Date of publication of application: **12.02.2020**
(21) Application number: 18781940.4
(22) Date of filing: 02.04.2018
(51) Int. Cl.: H01L 51/50, C07D 209/88, C09K 11/06, H05B 33/10

(54) **CHARGE-TRANSPORTING VARNISH**

(30) Priority: 05.04.2017 JP 2017074941
(71) Applicant: Nissan Chemical Corporation, Tokyo 103-6119 (JP)
(72) Inventor: NAKAIE Naoki, Funabashi-shi Chiba 274-0052 (JP)
(74) Representative: Mewburn Ellis LLP
(86) International application number: PCT/JP2018/014084
(87) International publication number: WO 2018/186340

(57) **Abstract**

Provided is a charge-transporting varnish comprising: a charge-transporting material such as an aniline derivative or a thiophene derivative; a fluorinated alkyl phosphate onium salt represented by the formula; and an organic solvent.

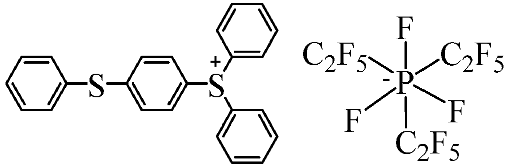

## Description

### TECHNICAL FIELD

The present invention relates to a charge-transporting varnish.

### BACKGROUND ART

In an organic electroluminescent (hereinafter, referred to as organic EL) device, a charge-transporting thin film made of an organic compound is used as a light-emitting layer and a charge injection layer. Particularly, a hole injection layer is responsible for a transfer of charges between a positive electrode and a hole injection layer or a light-emitting layer, and has an important function for achieving low-voltage driving and high brightness of an organic EL device.

A method of forming the hole injection layer is largely divided into a dry process represented by a vapor deposition method and a wet process represented by a spin coating method, and when these processes are compared, a thin film having a large area and high flatness can be efficiently produced by the method of the wet process. Therefore, as the area of the organic EL display is increased, a hole injection layer which can be formed by the wet process is currently desired.

In view of such circumstances, the present inventor has developed charge-transporting materials which are applicable to various wet processes and also if applied to a hole injection layer of an organic EL device, provide a thin film capable of realizing excellent EL device characteristics, and a compound having a good solubility in an organic solvent to be used (see for example, Patent Documents 1 to 4).

However, improvements have always been demanded for the wet process materials for the hole injection layer, and in particular, wet process materials which provide a thin film having excellent charge transportability have been demanded.

### PRIOR ART DOCUMENTS

### PATENT DOCUMENTS

Patent Document 1: WO 2008/032616
Patent Document 2: WO 2008/129947
Patent Document 3: WO 2006/025342
Patent Document 4: WO 2010/058777
Patent Document 5: JP-A 2014-205624

### SUMMARY OF INVENTION

### TECHNICAL PROBLEM

An object of the invention, which has been made under the above circumstances, is to provide a charge-transporting varnish which provides a charge-transporting thin film having excellent charge transportability, flatness, and uniformity with good reproducibility.

### SOLUTION TO PROBLEM

The present inventors have conducted an extensive investigation in order to achieve the above object, and as a result, have discovered that charge-transporting thin films of excellent charge transportability, flatness, and uniformity can be reproducibly obtained from a varnish prepared by dissolving a charge-transporting material and a specific fluorinated alkyl phosphate onium salt in an organic solvent, and that organic EL devices of excellent brightness characteristics can be obtained by using such a thin film as a hole-injecting layer, thereby completing the present invention.

That is, the present invention provides the following:
1. A charge-transporting varnish including a charge-transporting material, a fluorinated alkyl phosphate onium salt, and an organic solvent, wherein the fluorinated alkyl phosphate onium salt includes a fluorinated alkyl phosphate onium salt consisting of an anion of formula (a1) and a countercation: 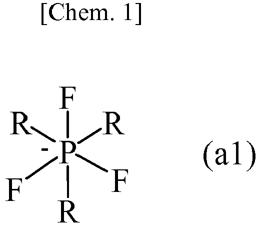 wherein R is an alkyl group of 1 to 10 carbon atoms, a fluoroalkyl group of 1 to 10 carbon atoms, an aralkyl group of 7 to 10 carbon atoms, or a fluoroaralkyl group of 7 to 10 carbon atoms.
2. The charge-transporting varnish of 1, wherein the charge-transporting material is at least one compound selected from the group consisting of aniline derivatives and thiophene derivatives.
3. The charge-transporting varnish of 2, wherein the charge-transporting material is an aniline derivative.
4. A charge-transporting thin film obtained from the charge-transporting varnish of any one of 1 to 3.
5. An organic electroluminescent device including the charge-transporting thin film of 4.
6. A method for producing a charge-transporting thin film, including steps of applying the charge-transporting varnish of any one of 1 to 3 onto a substrate, and evaporating off a solvent.

### ADVANTAGEOUS EFFECTS OF INVENTION

By using the charge-transporting varnish of the present invention, a charge-transporting thin film having excellent charge transportability, flatness, and uniformity can be obtained.

Further, the charge-transporting thin film having such characteristics can be suitably used as a thin film for an electronic device including an organic EL device. In particular, by applying the thin film to a hole injection layer of an organic EL device, an organic EL device with a low driving voltage can be obtained.

In addition, the charge-transporting varnish of the present invention can produce a thin film having excellent charge transportability with good reproducibility even when various wet processes allowing film formation on a large area, such as a spin coating method or a slit coating method, are used, and thus, can sufficiently respond to even the recent progress in the field of organic EL devices.

Further, since the thin film obtained from the charge-transporting varnish of the present invention has excellent charge transportability, and thus, it is expected to use the thin film as an antistatic film, a positive electrode buffer layer of an organic thin film solar cell, and the like.

### DESCRIPTION OF EMBODIMENTS

Hereinafter, the present invention is described in more detail.

The charge-transporting varnish according to the present invention includes a charge-transporting material, a fluorinated alkyl phosphate onium salt, and an organic solvent, in which the fluorinated alkyl phosphate onium salt includes a fluorinated alkyl phosphate onium salt consisting of an anion of formula (a1) and a countercation.

In addition, charge transportability is synonymous with electrical conductivity, and is also synonymous with hole transportability. Further, the charge-transporting varnish of the present invention may have charge transportability by itself, or a solid film obtained using the varnish may have charge transportability.

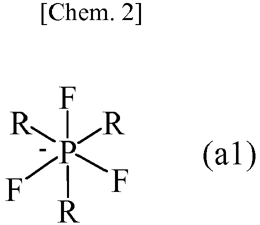

In formula (a1), R is an alkyl group of 1 to 10 carbon atoms, a fluoroalkyl group of 1 to 10 carbon atoms, an aralkyl group of 7 to 10 carbon atoms, or a fluoroaralkyl group of 7 to 10 carbon atoms.

The alkyl group of 1 to 10 carbon atoms may be linear, branched, or cyclic, and examples thereof include linear or branched alkyl groups of 1 to 10 carbon atoms such as a methyl group, an ethyl group, an n-propyl group, an isopropyl group, an n-butyl group, an isobutyl group, an s-butyl group, a t-butyl group, an n-pentyl group, an n-hexyl group, an n-heptyl group, an n-octyl group, an n-nonyl group, and an n-decyl group; and cyclic alkyl groups of 3 to 10 carbon atoms such as a cyclopropyl group, a cyclobutyl group, a cyclopentyl group, a cyclohexyl group, a cycloheptyl group, a cyclooctyl group, a cyclononyl group, a cyclodecyl group, a bicyclobutyl group, a bicyclopentyl group, a bicyclohexyl group, a bicycloheptyl group, a bicyclooctyl group, a bicyclononyl group, and a bicyclodecyl group; and the like, and an alkyl group of 1 to 8 carbon atoms is preferred, and an alkyl group of 1 to 6 carbon atoms is more preferred.

Examples of the aralkyl group of 7 to 10 carbon atoms include a group in which at least one of the hydrogen atoms of the alkyl group is substituted with an aryl group, for example, a benzyl group, a 1-naphthylmethylene group, a 2-naphthylmethylene group, a phenylethylene group, a 1-naphthylethylene group, a 2-naphthylmethylene group, and the like, and an aralkyl group of 7 to 9 carbon atoms is preferred.

Specific examples of the fluoroalkyl group of 1 to 10 carbon atoms include a group in which at least one of the hydrogen atoms of the alkyl group of 1 to 10 carbon atoms is substituted with a fluorine atom.

Specific examples thereof include a fluoromethyl group, a difluoromethyl group, a trifluoromethyl group, a pentafluoroethyl group, a 2,2,2-trifluoroethyl group, a heptafluoropropyl group, a 2,2,3,3,3-pentafluoropropyl group, a 2,2,3,3-tetrafluoropropyl group, a 2,2,2-trifluoro-1-(trifluoromethyl)ethyl group, a nonafluorobutyl group, a 4,4,4-trifluorobutyl group, an undecafluoropentyl group, a 2,2,3,3,4,4,5,5,5-nonafluoropentyl group, a 2,2,3,3,4,4,5,5-octafluoropentyl group, a tridecafluorohexyl group, a 2,2,3,3,4,4,5,5,6,6,6-undecafluorohexyl group, a 2,2,3,3,4,4,5,5,6,6-decafluorohexyl group, a 3,3,4,4,5,5,6,6,6-nonafluorohexyl group, and the like.

Specific examples of the fluoroaralkyl group of 7 to 10 carbon atoms include a group in which at least one of the hydrogen atoms of the aralkyl group of 7 to 10 carbon atoms is substituted with a fluorine atom.

Specific examples thereof include a perfluorobenzyl group, a pentafluorophenylmethylene group, a heptafluoro-1-naphthylmethylene group, a heptafluoro-2-naphthylmethylene group, a heptafluoro-1-naphthylethylene group, a heptafluoro-2-naphthylethylene group, and the like.

Examples of the anion of the above formula (a1) which can be suitably used in the present invention include those represented by the following formula, but are not limited thereto.

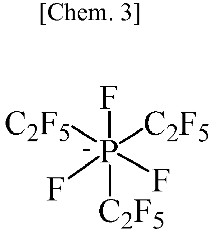

Meanwhile, the countercation is not particularly limited, but a cation of formula (c1) is preferred.

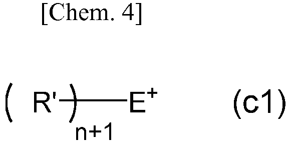

In formula (c1), E is an element having a valence of n of Groups 15 to 17, and thus, n is an integer of 1 to 3 corresponding to the valence of E.

n+1 R's are bonded to E, R' is independently a monovalent organic group, and two or more R's may be bonded to each other directly or via -O-, -S-, -SO-, -SO₂-, -NH-, -CO-, -COO-, -CONH-, an alkylene group, or a phenylene group to form a ring structure with the element E.

The monovalent organic group is not particularly limited, but is preferably an alkyl group of 1 to 20 carbon atoms, an alkenyl group of 2 to 20 carbon atoms, an alkynyl group of 2 to 20 carbon atoms, or an aryl group of 6 to 20 carbon atoms which may be substituted with Z, and more preferably an aryl group having 6 to 14 carbon atoms which may be substituted with Z.

The alkyl group of 1 to 20 carbon atoms may be linear, branched, or cyclic, and in addition to the groups represented above as an example of the alkyl group of 1 to 10 carbon atoms, examples of the alkyl group include an n-undecyl group, an n-dodecyl group, an n-tridecyl group, an n-tetradecyl group, an n-pentadecyl group, an n-hexadecyl group, an n-heptadecyl group, an n-octadecyl group, an n-nonadecyl group, an n-eicosanyl group, and the like, and an alkyl group of 1 to 18 carbon atoms is preferred, and an alkyl group of 1 to 8 carbon atoms is more preferred.

Specific examples of the alkenyl group having 2 to 20 carbon atoms include an ethenyl group, an n-1-propenyl group, an n-2-propenyl group, a 1-methylethenyl group, an n-1-butenyl group, an n-2-butenyl group, an n-3-butenyl group, a 2-methyl-1-propenyl group, a 2-methyl-2-propenyl group, a 1-ethylethenyl group, a 1-methyl-1-propenyl group, a 1-methyl-2-propenyl group, an n-1-pentenyl group, an n-1-decenyl group, an n-1-eicosenyl group, and the like.

Specific examples of the alkynyl group having 2 to 20 carbon atoms include an ethynyl group, an n-1-propynyl group, an n-2-propynyl group, an n-1-butynyl group, an n-2-butynyl group, an n-3-butynyl group, a 1-methyl-2-propynyl group, an n-1-pentynyl group, an n-2-pentynyl group, an n-3-pentynyl group, an n-4-pentynyl group, a 1-methyl-n-butynyl group, a 2-methyl-n-butynyl group, a 3-methyl-n-butynyl group, a 1,1-dimethyl-n-propynyl group, an n-1-hexynyl group, an n-1-decynyl group, an n-1-pentadecynyl group, an n-1-eicosynyl group, and the like.

Specific examples of the aryl group of 6 to 20 carbon atoms include a phenyl group, a 1-naphthyl group, a 2-naphthyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group, and the like, and an aryl group of 6 to 14 carbon atoms is preferred.

Z is an alkyl group of 1 to 20 carbon atoms, a haloalkyl group of 1 to 8 carbon atoms, an alkenyl group of 2 to 20 carbon atoms, an alkynyl group of 2 to 20 carbon atoms, an aryl group of 6 to 20 carbon atoms, a heteroaryl group of 2 to 20 carbon atoms, a nitro group, a hydroxyl group, a cyano group, an alkoxy group of 1 to 8 carbon atoms, an aryloxy group of 6 to 20 carbon atoms, an acyl group of 1 to 20 carbon atoms, an acyloxy group of 1 to 20 carbon atoms, an alkylthio group of 1 to 8 carbon atoms, an arylthio group of 6 to 20 carbon atoms, an alkylamino group of 1 to 8 dicarbon atoms, an arylamino group of 6 to 20 dicarbon atoms, an amino group, or a halogen atom.

Examples of these alkyl group, alkenyl group, alkynyl group, and aryl group include those as described above.

Examples of the halogen atom include a fluorine atom, a chlorine atom, a bromine atom, an iodine atom, and the like, and a fluorine atom is preferred.

Specific examples of the heteroaryl group having 2 to 20 carbon atoms include an oxygen-containing heteroaryl group such as a 2-thienyl group, a 3-thienyl group, a 2-furanyl group, a 3-furanyl group, a 2-oxazolyl group, a 4-oxazolyl group, a 5-oxazolyl group, a 3-isoxazolyl group, a 4-isoxazolyl group, and a 5-isoxazolyl group; a sulfur-containing heteroaryl group such as a 2-thiazolyl group, a 4-thiazolyl group, a 5-thiazolyl group, a 3-isothiazolyl group, a 4-isothiazolyl group, and a 5-isothiazolyl group; a nitrogen-containing heteroaryl group such as a 2-imidazolyl group, a 4-imidazolyl group, a 2-pyridyl group, a 3-pyridyl group, a 4-pyridyl group, a 2-pyrazyl group, a 3-pyrazyl group, a 5-pyrazyl group, a 6-pyrazyl group, a 2-pyrimidyl group, a 4-pyrimidyl group, a 5-pyrimidyl group, a 6-pyrimidyl group, a 3-pyridazyl group, a 4-pyridazyl group, a 5-pyridazyl group, a 6-pyridazyl group, a 1,2,3-triazin-4-yl group, a 1,2,3-triazin-5-yl group, a 1,2,4-triazin-3-yl group, a 1,2,4-triazin-5-yl group, a 1,2,4-triazin-6-yl group, a 1,3,5-triazin-2-yl group, a 1,2,4,5-tetrazin-3-yl group, a 1,2,3,4-tetrazin-5-yl group, a 2-quinolinyl group, a 3-quinolinyl group, a 4-quinolinyl group, a 5-quinolinyl group, a 6-quinolinyl group, a 7-quinolinyl group, a 8-quinolinyl group, a 1-isoquinolinyl group, a 3-isoquinolinyl group, a 4-isoquinolinyl group, a 5-isoquinolinyl group, a 6-isoquinolinyl group, a 7-isoquinolinyl group, a 8-isoquinolinyl group, a 2-quinoxanyl group, a 5-quinoxanyl group, a 6-quinoxanyl group, a 2-quinazolinyl group, a 4-quinazolinyl group, a 5-quinazolinyl group, a 6-quinazolinyl group, a 7-quinazolinyl group, a 8-quinazolinyl group, a 3-cinnolinyl group, a 4-cinnolinyl group, a 5-cinnolinyl group, a 6-cinnolinyl group, a 7-cinnolinyl group, and a 8-cinnolinyl group; and the like.

Specific examples of the haloalkyl group of 1 to 8 carbon atoms include a group in which at least one of the hydrogen atoms of the alkyl group of 1 to 8 carbon atoms among the alkyl groups represented above as an example is substituted with a halogen atom, and the like. In addition, the halogen atom may be any one of chlorine, bromine, iodine, and fluorine atoms. Among them, a fluoroalkyl group is preferred, and a perfluoroalkyl group is more preferred.

Specific examples of the fluoroalkyl group include those as described above.

Specific examples of the alkoxy group of 1 to 8 carbon atoms include a methoxy group, an ethoxy group, an n-propoxy group, an i-propoxy group, a c-propoxy group, an n-butoxy group, an i-butoxy group, an s-butoxy group, a t-butoxy group, an n-pentoxy group, an n-hexoxy group, an n-heptyloxy group, an n-octyloxy group, and the like.

Specific examples of the aryloxy group of 6 to 20 carbon atoms include a phenoxy group, a 1-naphthyloxy group, a 2-naphthyloxy group, a 1-anthryloxy group, a 2-anthryloxy group, a 9-anthryloxy group, a 1-phenanthryloxy group, a 2-phenanthryloxy group, a 3-phenanthryloxy group, a 4-phenanthryloxy group, a 9-phenanthryloxy group, and the like.

Specific examples of the acyl group of 1 to 20 carbon atoms include a formyl group, an acetyl group, a propionyl group, a butyryl group, an isobutyryl group, a valeryl group, an isovaleryl group, a benzoyl group, and the like.

Specific examples of the acyloxy group of 1 to 20 carbon atoms include a formyloxy group, an acetoxy group, a propionyloxy group, a butyryloxy group, an isobutyryloxy group, a valeryloxy group, an isovaleryloxy group, a benzoyloxy group, and the like.

Specific examples of the alkylthio group of 1 to 8 carbon atoms include a methylthio group, an ethylthio group, an n-propylthio group, an isopropylthio group, an n-butylthio group, an isobutylthio group, an s-butylthio group, a t-butylthio group, an n-pentylthio group, an n-hexylthio group, an n-heptylthio group, an n-octylthio group, and the like.

Specific examples of the arylthio group of 6 to 20 carbon atoms include a phenylthio group, a 1-naphthylthio group, a 2-naphthylthio group, a 1-anthrylthio group, a 2-anthrylthio group, a 9-anthrylthio group, a 1-phenanthrylthio group, a 2-phenanthrylthio group, a 3-phenanthrylthio group, a 4-phenanthrylthio group, a 9-phenanthrylthio group, and the like.

Specific examples of the alkylamino group of 1 to 8 dicarbon atoms include a dimethylamino group, a diethylamino group, a di-n-propylamino group, a di-i-propylamino group, a di-n-butylamino group, a di-i-butylamino group, a di-n-pentylamino group, a di-n-hexylamino group, a di-n-heptylamino group, a di-n-octylamino group, a methylethylamino group, and the like.

Specific examples of the arylamino group of 6 to 20 carbon atoms include a diphenylamino group, a 1-naphthylphenylamino group, a di(1-naphthyl)amino group, a 1-naphthyl-2-naphthylamino group, a di(2-naphthyl)amino group, and the like.

As E, particularly O (oxygen), N (nitrogen), P (phosphorus), S (sulfur), or I (iodine) is preferred, among Groups 15 to 17 elements, and especially, S, I, N, and P giving an onium ion which is stable and easy to handle are more preferred, and S and I are still more preferred. Corresponding onium ions are oxonium, ammonium, phosphonium, sulfonium, and iodonium.

Specific examples of the onium ion represented by (R')ₙ₊₁-E⁺ include the following, but are not limited thereto.

Specific examples of the oxonium ion include oxoniums such as trimethyloxonium, diethylmethyloxonium, triethyloxonium, and tetramethylenemethyloxonium; pyryliums such as 4-methylpyrylium, 2,4,6-trimethylpyrylium, 2,6-di-t-butylpyrylium, and 2,6-diphenylpyrylium; chromeniums and isochromeniums such as 2,4-dimethylchromenium and 1,3-dimethylisochromenium; and the like.

Specific examples of the ammonium ion include tetraalkylammoniums such as tetramethylammonium, ethyltrimethylammonium, diethyldimethylammonium, triethylmethylammonium, and tetraethylammonium; pyrrolidiniums such as N,N-dimethylpyrrolidinium, N-ethyl-N-methylpyrrolidinium, and N,N-diethylpyrrolidinium; imidazoliniums such as N,N'-dimethylimidazolinium, N,N'-diethylimidazolinium, N-ethyl-N'-methylimidazolinium, 1,3,4-trimethylimidazolinium, and 1,2,3,4-tetramethylimidazolinium; tetrahydropyrimidiniums such as N,N'-dimethyltetrahydropyrimidinium; morpholiniums such as N,N'-dimethylmorpholinium; piperidiniums such as N,N'-diethylpiperidinium; pyridiniums such as N-methylpyridinium, N-benzylpyridinium, and N-phenacylpyridium; imidazoliums such as N,N'-dimethylimidazolium; quinoliums such as N-methylquinolium, N-benzylquinolium, and N-phenacylquinolium; isoquinoliums such as N-methylisoquinolium; thiazoniums such as benzylbenzothiazonium and phenacylbenzothiazonium; and acridiums such as benzylacridium and phenacylacridium.

Specific examples of the phosphonium ion include tetraarylphosphoniums such as tetraphenylphosphonium, tetra-p-tolyl phosphonium, tetrakis(2-methoxyphenyl)phosphonium, tetrakis(3-methoxyphenyl)phosphonium, and tetrakis(4-methoxyphenyl)phosphonium; triarylphosphoniums such as triphenylbenzylphosphonium, triphenylphenacylphosphonium, triphenylmethylphosphonium, and triphenylbutylphosphonium; and tetraalkylphosphoniums such as triethylbenzylphosphonium, tributylbenzylphosphonium, tetraethylphosphonium, tetrabutylphosphonium, tetrahexylphosphonium, triethylphenacylphosphonium, and tributylphenacylphosphonium.

Specific examples of the sulfonium ions include triarylsulfoniums such as triphenylsulfonium, tri-p-tolylsulfonium, tri-o-tolylsulfonium, tris(4-methoxyphenyl)sulfonium, 1-naphthyl diphenylsulfonium, 2-naphthyl diphenylsulfonium, tris(4-fluorophenyl)sulfonium, tri-1-naphthylsulfonium, tri-2-naphthylsulfonium, tris(4-hydroxyphenyl)sulfonium, 4-(phenylthio)phenyl diphenylsulfonium, 4-(p-tolylthio)phenyl di-p-tolylsulfonium, 4-(4-methoxyphenylthio)phenyl bis(4-methoxyphenyl)sulfonium, 4-(phenylthio)phenyl bis(4-fluorophenyl)sulfonium, 4-(phenylthio)phenyl bis(4-methoxyphenyl)sulfonium, 4-(phenylthio)phenyl di-p-tolylsulfonium, [4-(4-biphenylylthio)phenyl]-4-biphenylylphenylsulfonium, [4-(2-thioxanthonylthio)phenyl]diphenylsulfonium, bis[4-(diphenylsulfonio)phenyl]sulfide, bis[4-{bis[4-(2-hydroxyethoxy)phenyl]sulfonio}phenyl]sulfide, bis{4-[bis(4-fluorophenyl)sulfonio]phenyl}sulfide, bis{4-[bis(4-methylphenyl)sulfonio]phenyl}sulfide, bis{4-[bis(4-methoxyphenyl)sulfonio]phenyl}sulfide, 4-(4-benzoyl-2-chlorophenylthio)phenyl bis(4-fluorophenyl)sulfonium, 4-(4-benzoyl-2-chlorophenylthio)phenyl diphenylsulfonium, 4-(4-benzoylphenylthio)phenylbis(4-fluorophenyl)sulfonium, 4-(4-benzoylphenylthio)phenyl diphenylsulfonium, 7-isopropyl-9-oxo-10-thia-9,10-dihydroanthracene-2-yl di-p-tolylsulfonium, 7-isopropyl-9-oxo-10-thia-9,10-dihydroanthracene-2-yl diphenylsulfonium, 2-[(di-p-tolyl)sulfonio]thioxanthone, 2-[(diphenyl)sulfonio]thioxanthone, 4-(9-oxo-9H-thioxanthen-2-yl)thiophenyl-9-oxo-9H-thioxanthen-2-ylphenylsulfonium, 4-[4-(4-t-butylbenzoyl)phenylthio]phenyl di-p-tolylsulfonium, 4-[4-(4-t-butylbenzoyl)phenylthio]phenyl diphenylsulfonium, 4-[4-(benzoylphenylthio)]phenyl di-p-tolylsulfonium, 4-[4-(benzoylphenylthio)]phenyl diphenylsulfonium, 5-(4-methoxyphenyl)thiaanthrenium, 5-phenylthiaanthrenium, 5-tolylthiaanthrenium, 5-(4-ethoxyphenyl)thiaanthrenium, and 5-(2,4,6-trimethylphenyl)thiaanthrenium; diarylsulfoniums such as diphenylphenacylsulfonium, diphenyl 4-nitrophenacylsulfonium, diphenylbenzylsulfonium, and diphenylmethylsulfonium; monoarylsulfoniums such as phenylmethylbenzylsulfonium, 4-hydroxyphenylmethylbenzylsulfonium, 4-methoxyphenylmethylbenzylsulfonium, 4-acetocarbonyloxyphenylmethylbenzylsulfonium, 4-hydroxyphenyl(2-naphthylmethyl)methylsulfonium, 2-naphthylmethylbenzylsulfonium, 2-naphthylmethyl(1-ethoxycarbonyl)ethylsulfonium, phenylmethylphenacylsulfonium, 4-hydroxyphenylmethylphenacylsulfonium, 4-methoxyphenylmethylphenacylsulfonium, 4-acetocarbonyloxyphenylmethylphenacylsulfonium, 2-naphthylmethylphenacylsulfonium, 2-naphthyloctadecylphenacylsulfonium, and 9-anthracenylmethylphenacylsulfonium; and tri alkylsulfoniums such as dimethylphenacylsulfonium, phenacyltetrahydrothiophenium, dimethylbenzylsulfonium, benzyltetrahydrothiophenium, and octadecylmethylphenacylsulfonium; and the like.

Specific examples of the iodonium ion include diphenyliodonium, di-p-tolyliodonium, bis(4-dodecylphenyl)iodonium, bis(4-methoxyphenyl)iodonium, (4-octyloxyphenyl)phenyliodonium, bis(4-decyloxy)phenyliodonium, 4-(2-hydroxytetradecyloxy)phenylphenyliodonium, 4-isopropylphenyl(p-tolyl)iodonium, 4-isobutylphenyl(p-tolyl)iodonium, and the like.

In the present invention, the fluorinated alkyl phosphate onium salt may be used alone or in combination of two or more.

Further, if necessary, other known fluorinated alkyl phosphate onium salts may be used in combination.

In addition, the fluorinated alkyl phosphate onium salt can be synthesized by a known method described in, for example, JP-A 2012-246456 and the like.

The fluorinated alkyl phosphate onium salt may be previously dissolved in an organic solvent, in order to facilitate dissolution in the charge-transporting varnish.

Examples of such an organic solvent include carbonates such as propylene carbonate, ethylene carbonate, 1,2-butylene carbonate, dimethyl carbonate, and diethyl carbonate; ketones such as acetone, methyl ethyl ketone, cyclohexanone, methyl isoamyl ketone, and 2-heptanone; polyvalent alcohols and derivatives thereof such as monomethyl ether, monoethyl ether, monopropyl ether, monobutyl ether, or monophenyl ether of ethylene glycol, ethylene glycol monoacetate, diethylene glycol, diethylene glycol monoacetate, propylene glycol, propylene glycol monoacetate, dipropylene glycol, or dipropylene glycol monoacetate; cyclic ethers such as dioxane; esters such as ethyl formate, methyl lactate, ethyl lactate, methyl acetate, ethyl acetate, butyl acetate, methyl pyruvate, methyl acetoacetate, ethyl acetoacetate, ethyl pyruvate, ethyl ethoxyacetate, methyl methoxypropionate, ethyl ethoxypropionate, methyl 2-hydroxypropionate, ethyl 2-hydroxypropionate, ethyl 2-hydroxy-2-methylpropionate, methyl 2-hydroxy-3-methylbutanate, 3-methoxybutyl acetate, and 3-methyl-3-methoxybutyl acetate; aromatic hydrocarbons such as toluene, xylene, 3-phenoxytolulene, and 4-methoxytoluene; and the like, and these solvents may be used alone or in combination of two or more.

When the organic solvent is used, the use ratio thereof is preferably 15 to 1,000 parts by weight, and more preferably 30 to 500 parts by weight, per 100 parts by weight of the fluorinated alkyl phosphate onium salt.

The charge-transporting material used in the present invention is not particularly limited, but can be suitably selected from those conventionally known in the fields of organic EL and the like and used.

Specific examples thereof include various hole-transporting materials, for example, arylamine derivatives such as an oligoaniline derivative, an N,N'-diarylbenzidine derivative, and an N,N,N',N'-tetraaryl benzidine derivative; thiophene derivatives such as an oligothiophene derivative, a thienothiophene derivative, and a thienobenzothiophene derivative; pyrrole derivatives such as oligopyrrole, and among them, an arylamine derivative and a thiophene derivative are preferred, an arylamine derivative is more preferred, and the aniline derivative represented by formula (1) or (2) is still more preferred.

Further, a molecular weight of the charge-transporting material is not particularly limited, but from a viewpoint of preparing a uniform varnish providing a thin film having high flatness, the molecular weight is preferably 200 to 9,000, from a viewpoint of obtaining charge transportability with high solvent resistance, the molecular weight is more preferably 300 or more, still more preferably 400 or more, and from a viewpoint of preparing a uniform varnish providing a thin film having high flatness with good reproducibility, the molecular weight is more preferably 8,000 or less, still more preferably 7,000 or less, further preferably 6,000 or less, and most preferably 5,000 or less.

In addition, from a viewpoint of preventing the charge-transporting material from being separated when formed into a thin film, it is preferred that the charge-transporting material has no molecular weight distribution (dispersion degree 1) (that is, a single molecular weight is preferred).

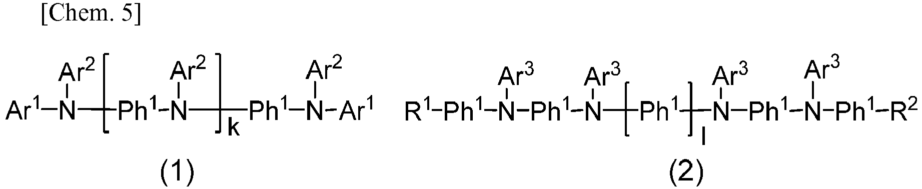

In the above formula (2), R¹ and R², independently of each other, represent a hydrogen atom, a halogen atom, a nitro group, a cyano group, or an alkyl group having 1 to 20 carbon atoms, an alkenyl group having 2 to 20 carbon atoms, an alkynyl group having 2 to 20 carbon atoms, an aryl group having 6 to 20 carbon atoms, or a heteroaryl group having 2 to 20 carbon atoms, which may be substituted by a halogen atom, and specific examples thereof include those as described for the above formula (c1).

Among them, R¹ and R² are preferably a hydrogen atom, a fluorine atom, a cyano group, an alkyl group having 1 to 20 carbon atoms which may be substituted by a halogen atom, an aryl group having 6 to 20 carbon atoms which may be substituted by a halogen atom, or a heteroaryl group having 2 to 20 carbon atoms which may be substituted by a halogen atom, more preferably a hydrogen atom, a fluorine atom, a cyano group, an alkyl group having 1 to 10 carbon atoms which may be substituted by a halogen atom, or a phenyl group which may be substituted by a halogen atom, still more preferably a hydrogen atom, a fluorine atom, a methyl group, or a trifluoromethyl group, and most preferably a hydrogen atom.

Ph¹ in the above formulae (1) and (2) represents a group represented by formula (PI).

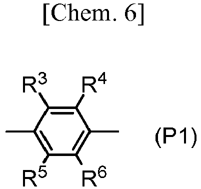

Wherein R³ to R⁶, independently of one another, represent a hydrogen atom, a halogen atom, a nitro group, a cyano group, or an alkyl group having 1 to 20 carbon atoms, an alkenyl group having 2 to 20 carbon atoms, an alkynyl group having 2 to 20 carbon atoms, an aryl group having 6 to 20 carbon atoms, or a heteroaryl group having 2 to 20 carbon atoms, which may be substituted by halogen atom, and specific examples thereof include those as described for the above formula (c1).

In particular, R³ to R⁶ are preferably a hydrogen atom, a fluorine atom, a cyano group, an alkyl group having 1 to 20 carbon atoms which may be substituted by a halogen atom, an aryl group having 6 to 20 carbon atoms which may be substituted by a halogen atom, or a heteroaryl group having 2 to 20 carbon atoms which may be substituted by a halogen atom, more preferably a hydrogen atom, a fluorine atom, a cyano group, an alkyl group having 1 to 10 carbon atoms which may be substituted by a halogen atom, or a phenyl group which may be substituted by a halogen atom, still more preferably a hydrogen atom, a fluorine atom, a methyl group, or a trifluoromethyl group, and most preferably a hydrogen atom.

Hereinafter, specific examples of the group suitable as Ph¹ include the following, but are not necessarily limited thereto.

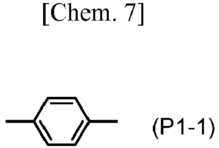

In the above formula (1), Ar¹'s, independently of each other, represent a group represented by any one of formulae (B1) to (B11), and in particular, a group represented by any one of formulae (B1') to (B11') is preferred.

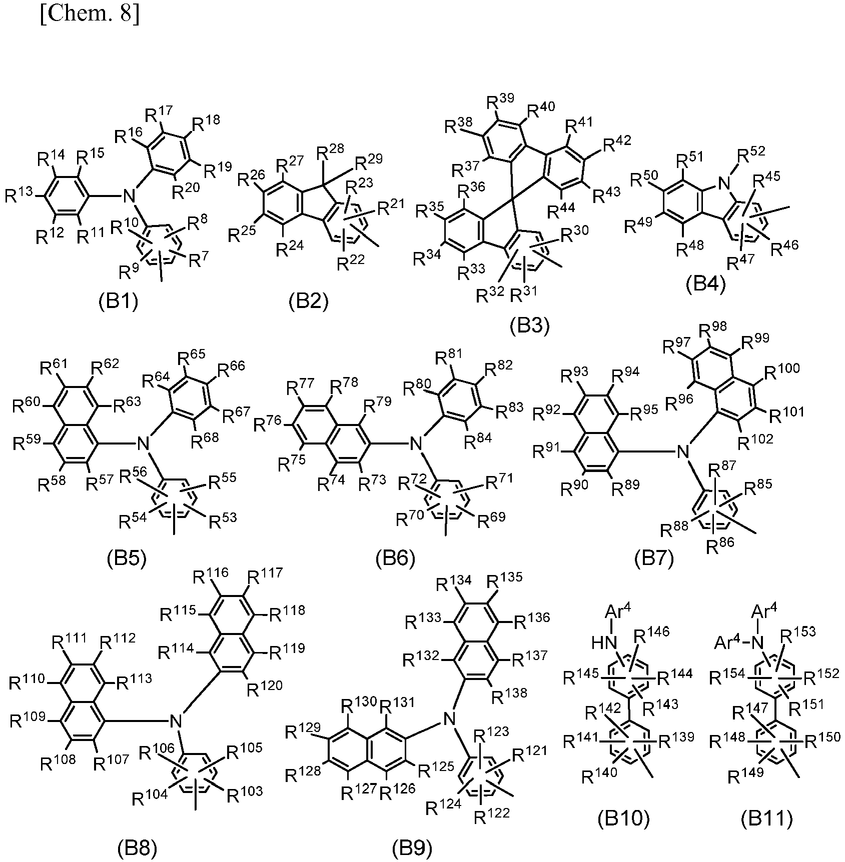

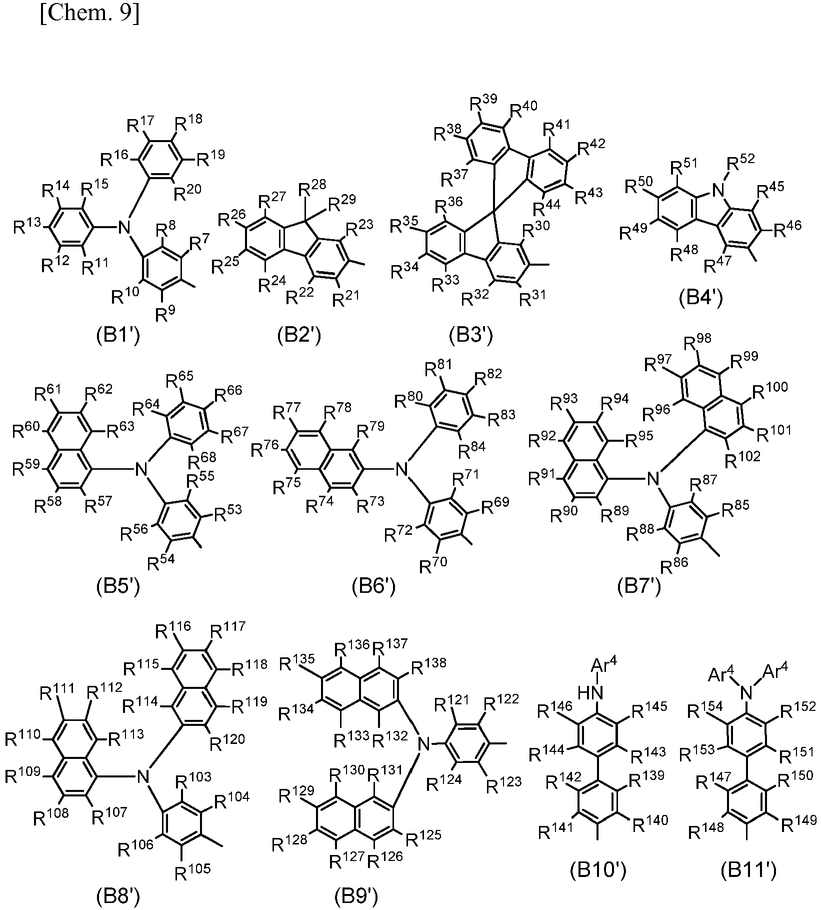

Wherein R⁷ to R²⁷, R³⁰ to R⁵¹, and R⁵³ to R¹⁵⁴, independently of one another, represent a hydrogen atom, a halogen atom, a nitro group, a cyano group, or a diphenylamino group, an alkyl group having 1 to 20 carbon atoms, an alkenyl group having 2 to 20 carbon atoms, an alkynyl group having 2 to 20 carbon atoms, an aryl group having 6 to 20 carbon atoms, or a heteroaryl group having 2 to 20 carbon atoms, which may be substituted by a halogen atom, R²⁸ and R²⁹, independently of each other, represent an aryl group having 6 to 20 carbon atoms or a heteroaryl group having 2 to 20 carbon atoms, which may be substituted by Z¹; R⁵² represents a hydrogen atom, or an alkyl group having 1 to 20 carbon atoms, an alkenyl group having 2 to 20 carbon atoms, or an alkynyl group having 2 to 20 carbon atoms, which may be substituted by Z⁴, or an aryl group having 6 to 20 carbon atoms or a heteroaryl group having 2 to 20 carbon atoms, which may be substituted by Z¹; Z¹ represents a halogen atom, a nitro group, a cyano group, or an alkyl group having 1 to 20 carbon atoms, an alkenyl group having 2 to 20 carbon atoms, or an alkynyl group having 2 to 20 carbon atoms, which may be substituted by Z²; Z² represents a halogen atom, a nitro group, a cyano group, or an aryl group having 6 to 20 carbon atoms or a heteroaryl group having 2 to 20 carbon atoms, which may be substituted by Z³; Z³ represents a halogen atom, a nitro group, or a cyano group; Z⁴ represents a halogen atom, a nitro group, a cyano group, or an aryl group having 6 to 20 carbon atoms or a heteroaryl group having 2 to 20 carbon atoms, which may be substituted by Z⁵; Z⁵ represents a halogen atom, a nitro group, a cyano group, or an alkyl group having 1 to 20 carbon atoms, an alkenyl group having 2 to 20 carbon atoms, or an alkynyl group having 2 to 20 carbon atoms, which may be substituted by Z³; and specific examples of these halogen atom, alkyl group having 1 to 20 carbon atoms, alkenyl group having 2 to 20 carbon atoms, alkynyl group having 2 to 20 carbon atoms, aryl group having 6 to 20 carbon atoms, and heteroaryl group having 2 to 20 carbon atoms include those as described in the above formula (c1).

In particular, R⁷ to R²⁷, R³⁰ to R⁵¹, and R⁵³ to R¹⁵⁴ are preferably a hydrogen atom, a fluorine atom, a cyano group, a diphenylamino group which may be substituted by a halogen atom, an alkyl group having 1 to 20 carbon atoms which may be substituted by a halogen atom, an aryl group having 6 to 20 carbon atoms which may be substituted by a halogen atom, or a heteroaryl group having 2 to 20 carbon atoms which may be substituted by a halogen atom, more preferably a hydrogen atom, a fluorine atom, a cyano group, an alkyl group having 1 to 10 carbon atoms which may be substituted by a halogen atom, or a phenyl group which may be substituted by a halogen atom, still more preferably a hydrogen atom, a fluorine atom, a methyl group, or a trifluoromethyl group, and most preferably a hydrogen atom.

In addition, R²⁸ and R²⁹ are preferably an aryl group having 6 to 14 carbon atoms which may be substituted by Z¹ or a heteroaryl group having 2 to 14 carbon atoms which may be substituted by Z¹, more preferably an aryl group having 6 to 14 carbon atoms which may be substituted by Z¹, and still more preferably a phenyl group which may be substituted by Z¹, a 1-naphthyl group which may be substituted by Z¹, or a 2-naphthyl group which may be substituted by Z¹.

In addition, R⁵² is preferably a hydrogen atom, an aryl group having 6 to 20 carbon atoms which may be substituted by Z¹, a heteroaryl group having 2 to 20 carbon atoms which may be substituted by Z¹, or an alkyl group having 1 to 20 carbon atoms which may be substituted by Z⁴, more preferably a hydrogen atom, an aryl group having 6 to 14 carbon atoms which may be substituted by Z¹, a heteroaryl group having 2 to 14 carbon atoms which may be substituted by Z¹, or an alkyl group having 1 to 10 carbon atoms which may be substituted by Z⁴, still more preferably a hydrogen atom, an aryl group having 6 to 14 carbon atoms which may be substituted by Z¹, a heteroaryl group having 2 to 14 carbon atoms which may be substituted by Z¹, or an alkyl group having 1 to 10 carbon atoms which may be substituted by Z⁴, and further preferably a hydrogen atom, a phenyl group which may be substituted by Z¹, a 1-naphthyl group which may be substituted by Z¹, a 2-naphthyl group which may be substituted by Z¹, a 2-pyridyl group which may be substituted by Z¹, a 3-pyridyl group which may be substituted by Z¹, a 4-pyridyl group which may be substituted by Z¹, or a methyl group which may be substituted by Z⁴.

In addition, Ar⁴'s, independently of each other, represent an aryl group having 6 to 20 carbon atoms which may be substituted by a arylamino group having 6 to 20 carbon atoms.

Specific examples of the aryl group having 6 to 20 carbon atoms and the arylamino group having 6 to 20 carbon atoms include those as described in formula (c1).

Ar⁴ is preferably a phenyl group, a 1-naphthyl group, a 2-naphthyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group, a p-(diphenylamino)phenyl group, a p-(1-naphthylphenylammo)phenyl group, a p-(di(1-naphthyl)amino)phenyl group, a p-(1-naphthyl-2-naphthylamino)phenyl group, a p-(di(2-naphthyl)amino)phenyl group, and more preferably a p-(diphenylamino)phenyl group.

Hereinafter, specific examples of the group suitable as Ar¹ include the following, but are not necessarily limited thereto. 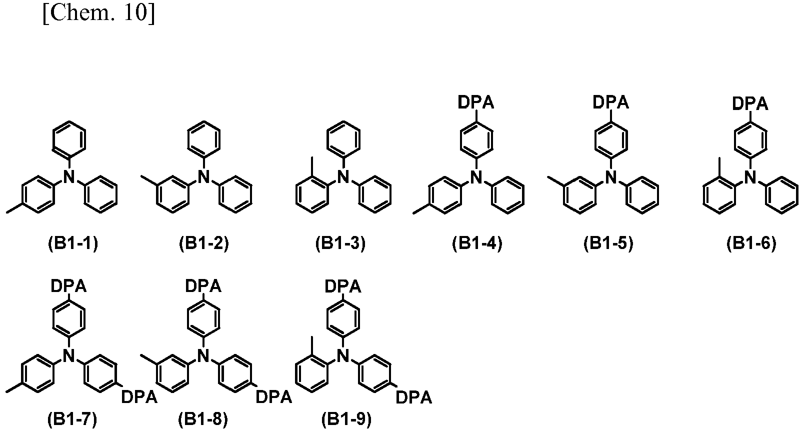 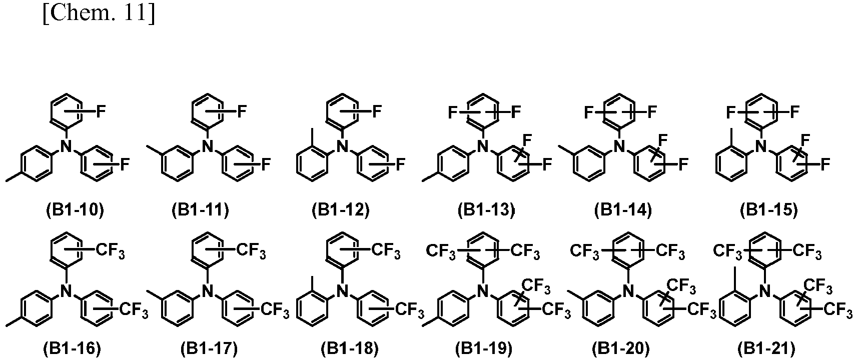 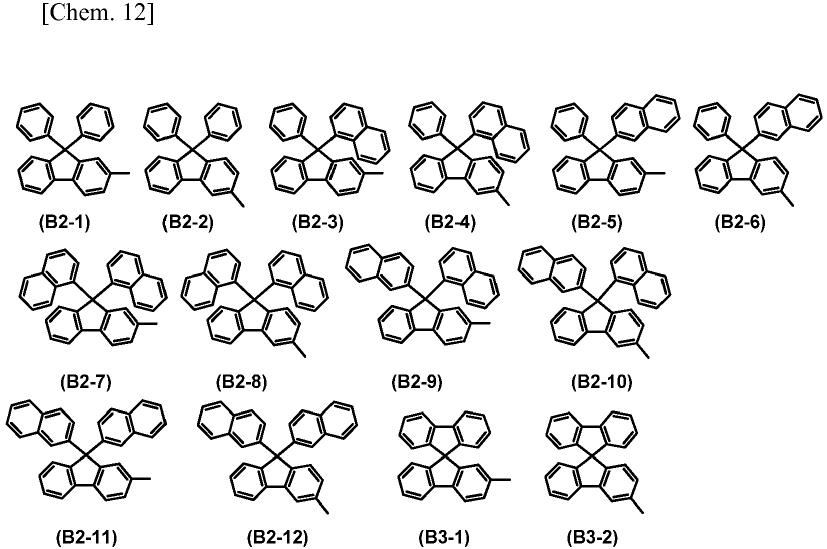 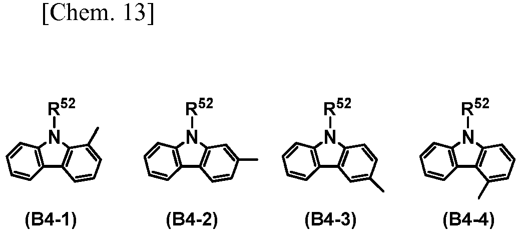 wherein R⁵² represents the same meaning as described above.

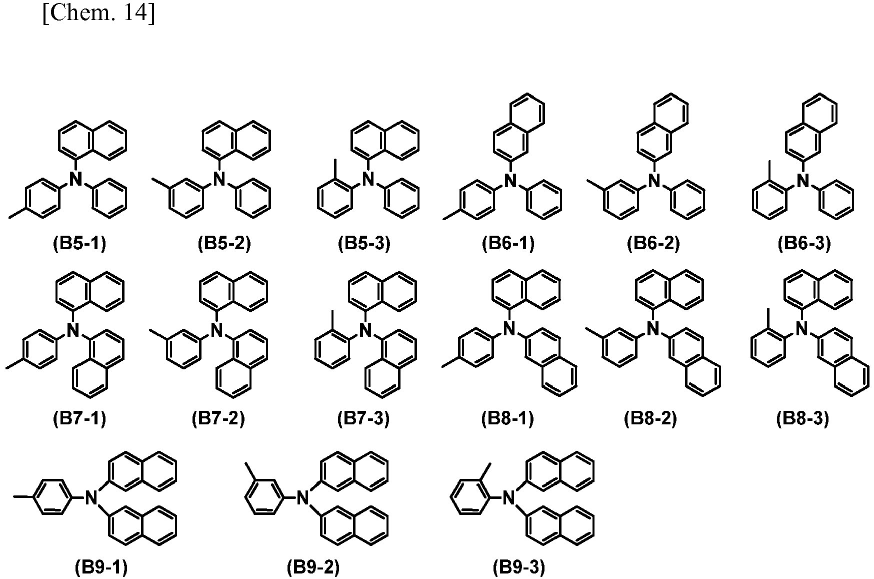

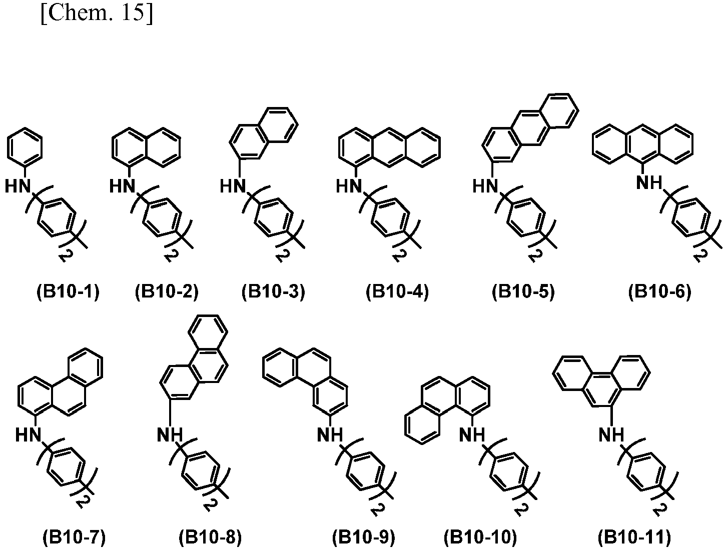

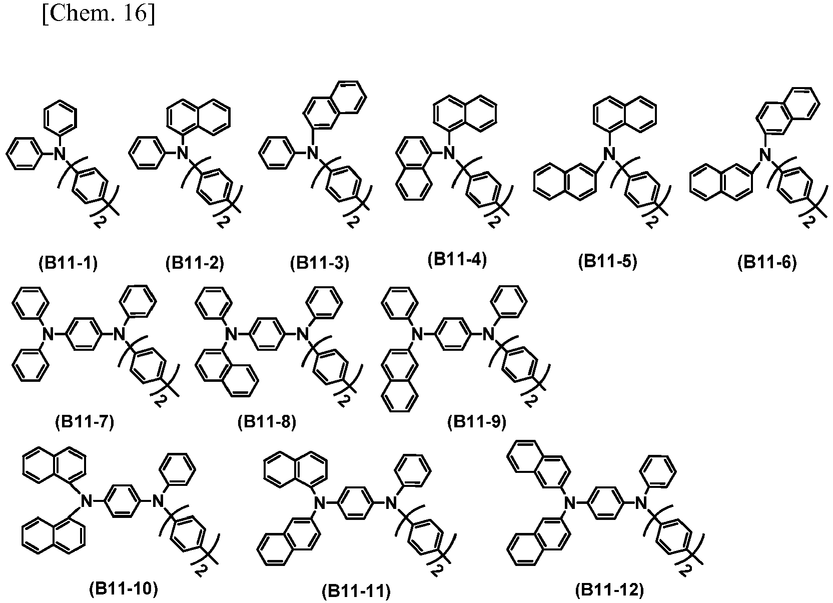

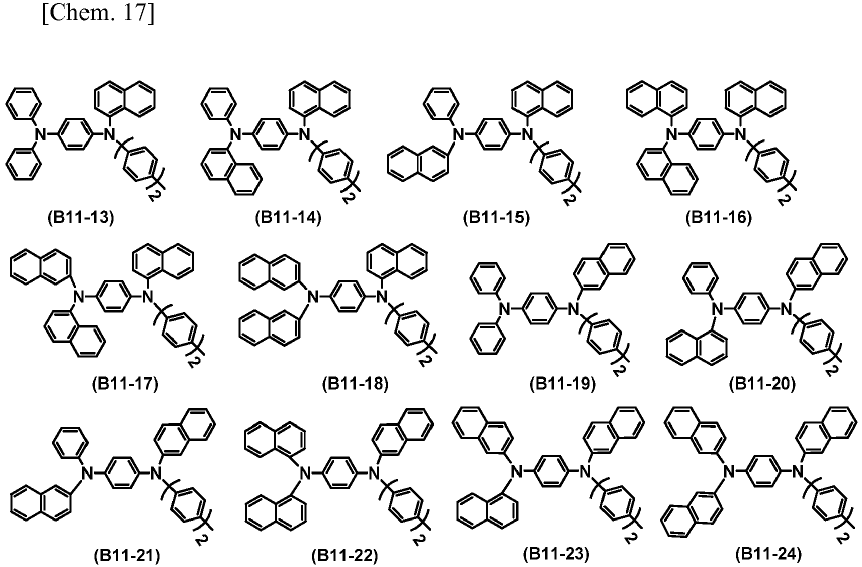

In the above formula (1), Ar²'s, independently of each other, represent a group represented by any one of formulae (A1) to (A18).

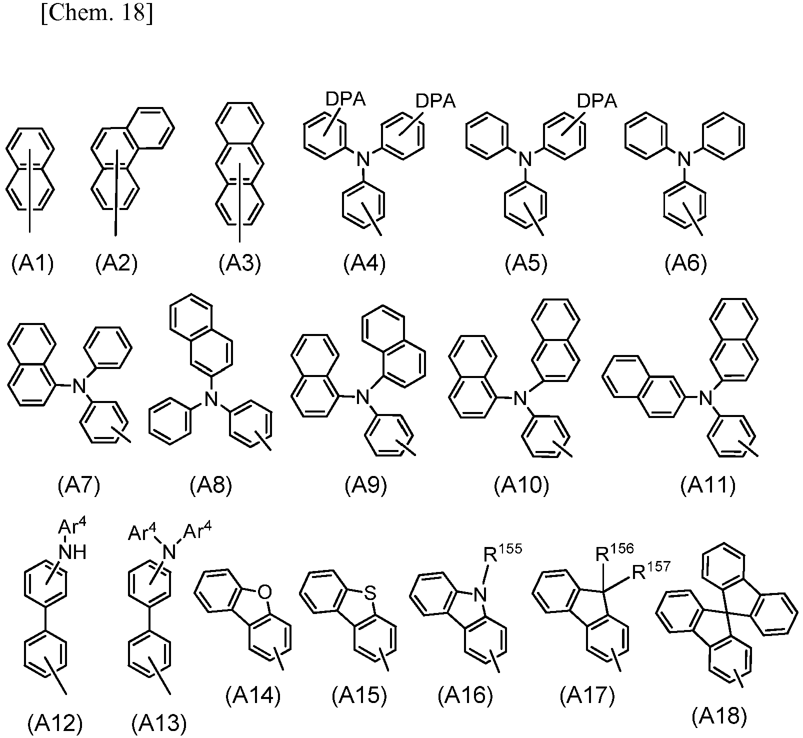

Here, in the formula, R¹⁵⁵ represents a hydrogen atom, or an alkyl group having 1 to 20 carbon atoms, an alkenyl group having 2 to 20 carbon atoms, or an alkynyl group having 2 to 20 carbon atoms, which may be substituted by Z⁴, or an aryl group having 6 to 20 carbon atoms or a heteroaryl group having 2 to 20 carbon atoms, which may be substituted by Z¹, R¹⁵⁶ and R¹⁵⁷, independently of each other, represent an aryl group having 6 to 20 carbon atoms or a heteroaryl group having 2 to 20 carbon atoms which may be substituted by Z¹, DPA represents a diphenylamino group, and Ar⁴, Z¹, and Z³ to Z⁵ represent the same meaning as described above. Specific examples of these halogen atom, alkyl group having 1 to 20 carbon atoms, alkenyl group having 2 to 20 carbon atoms, alkynyl group having 2 to 20 carbon atoms, aryl group having 6 to 20 carbon atoms, and heteroaryl group having 2 to 20 carbon atoms include those as described for the above formula (c1).

In particular, R¹⁵⁵ is preferably a hydrogen atom, an aryl group having 6 to 20 carbon atoms which may be substituted by Z¹, a heteroaryl group having 2 to 20 carbon atoms which may be substituted by Z¹, or an alkyl group having 1 to 20 carbon atoms which may be substituted by Z⁴, more preferably a hydrogen atom, an aryl group having 6 to 14 carbon atoms which may be substituted by Z¹, a heteroaryl group having 2 to 14 carbon atoms which may be substituted by Z¹, or an alkyl group having 1 to 10 carbon atoms which may be substituted by Z⁴, still more preferably a hydrogen atom, an aryl group having 6 to 14 carbon atoms which may be substituted by Z¹, a heteroaryl group having 2 to 14 carbon atoms which may be substituted by Z¹, or an alkyl group having 1 to 10 carbon atoms which may be substituted by Z⁴, and further preferably a hydrogen atom, a phenyl group which may be substituted by Z¹, a 1-naphthyl group which may be substituted by Z¹, a 2-naphthyl group which may be substituted by Z¹, a 2-pyridyl group which may be substituted by Z¹, a 3-pyridyl group which may be substituted by Z¹, a 4-pyridyl group which may be substituted by Z¹, or a methyl group which may be substituted by Z⁴.

In addition, R¹⁵⁶ and R¹⁵⁷ are preferably an aryl group having 6 to 14 carbon atoms which may be substituted by Z¹ or a heteroaryl group having 2 to 14 carbon atoms which may be substituted by Z¹, more preferably an aryl group having 6 to 14 carbon atoms which may be substituted by Z¹, and still more preferably a phenyl group which may be substituted by Z¹, a 1-naphthyl group which may be substituted by Z¹, or a 2-naphthyl group which may be substituted by Z¹.

Hereinafter, specific examples of the group suitable as Ar² include the following, but are not necessarily limited thereto. 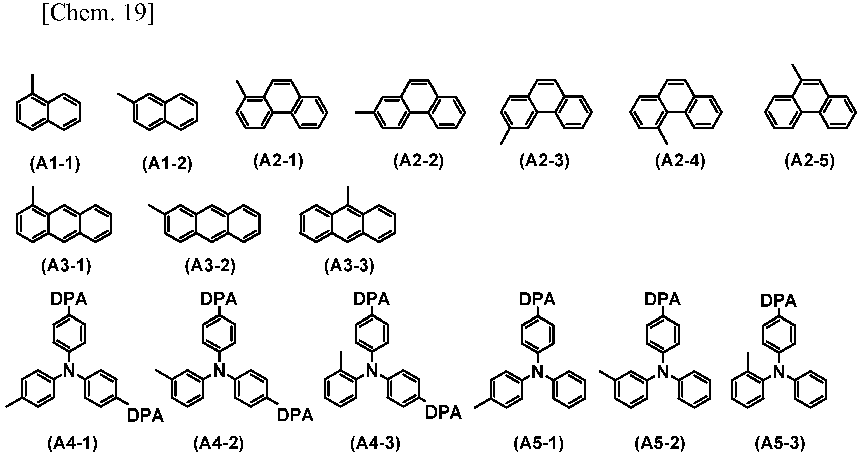 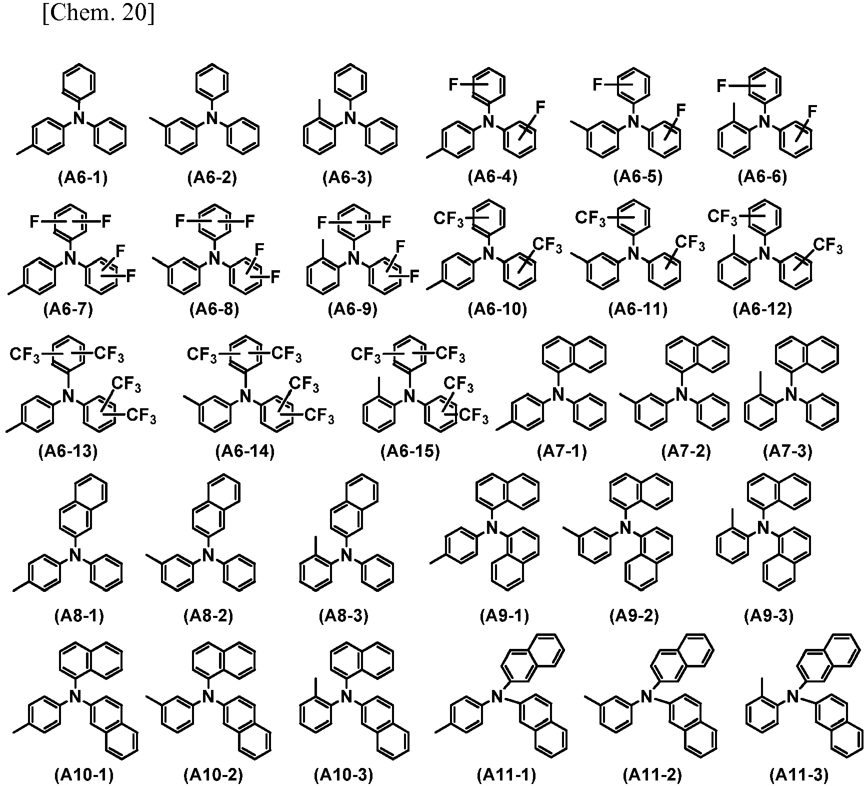 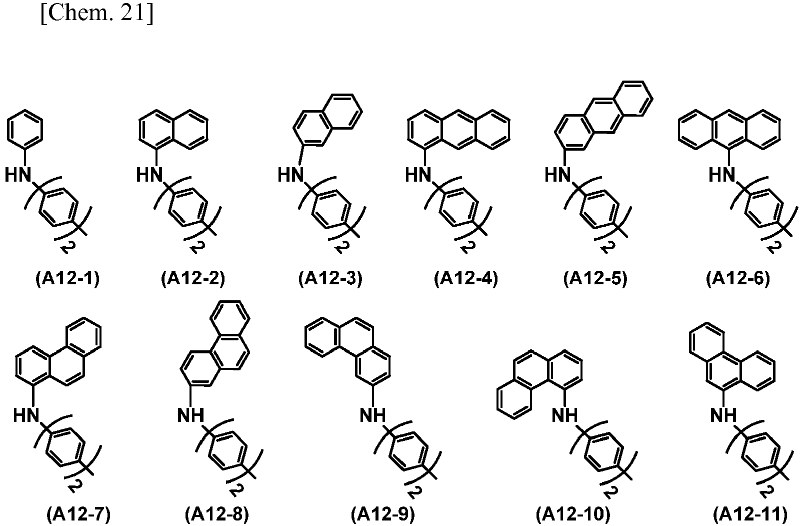 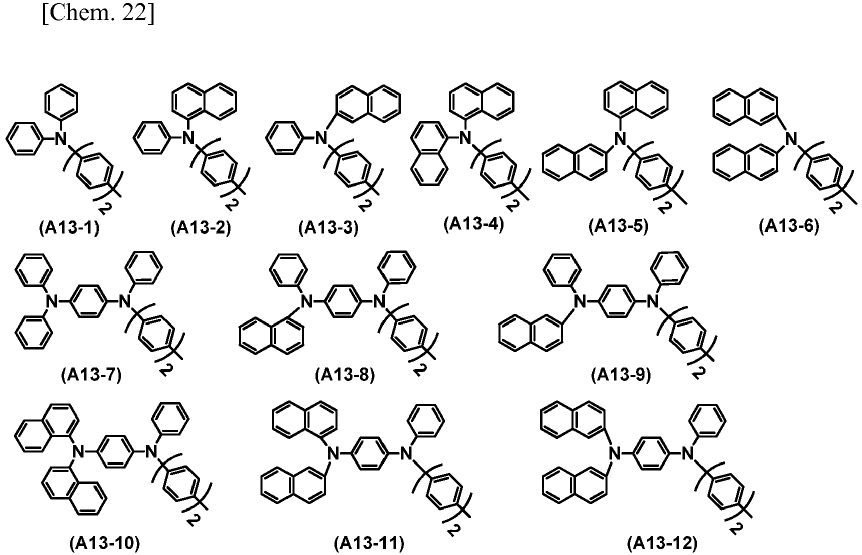 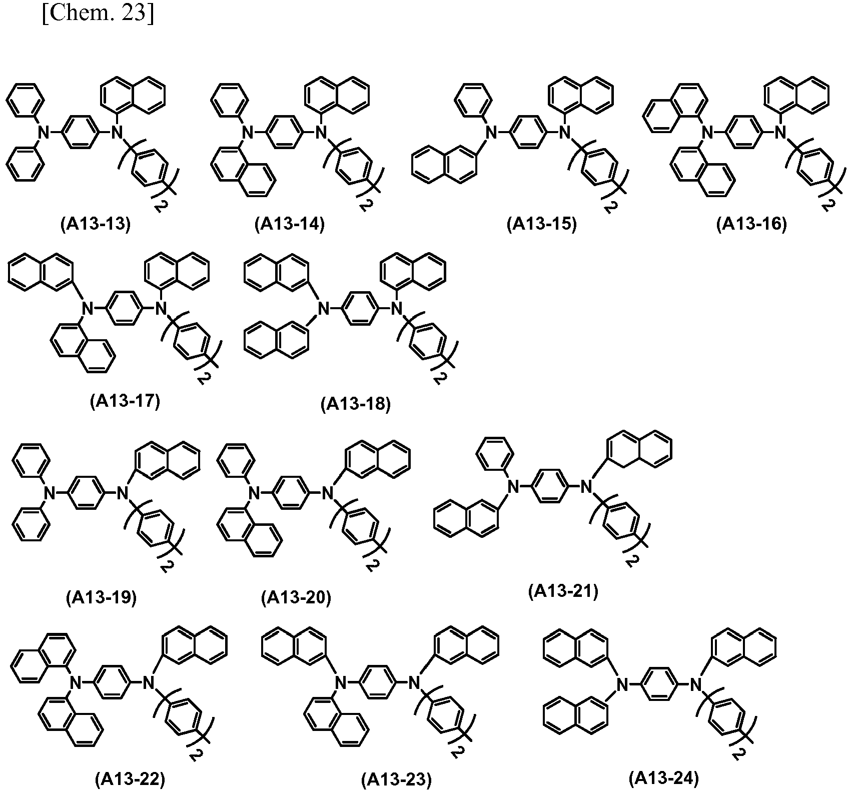 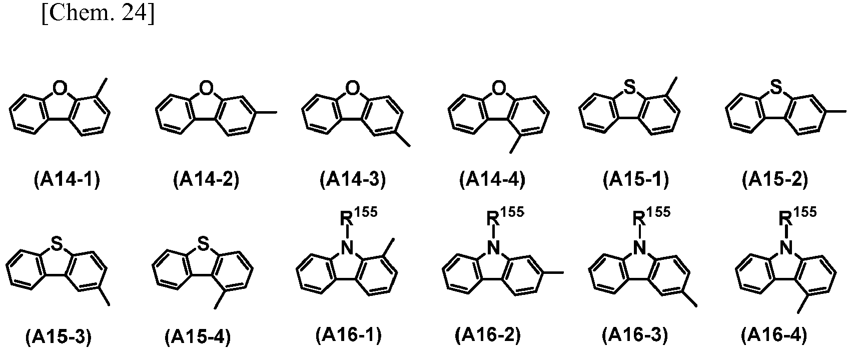 wherein R¹⁵⁵ represents the same meaning as described above.

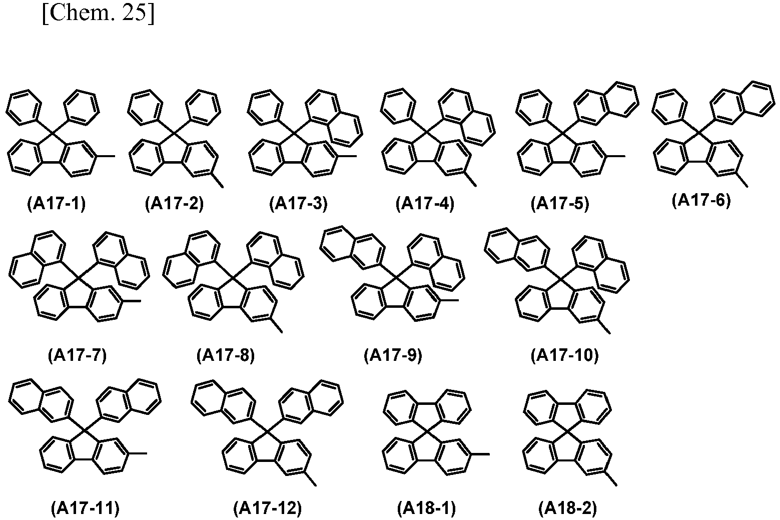

In addition, in formula (1), considering ease of synthesis of the resulting aniline derivative, it is preferred that all Ar¹'s are the same group and all Ar²'s are the same group, and it is more preferred that all Ar¹'s and Ar²'s are the same group. That is, the aniline derivative represented by formula (1) is more preferably the aniline derivative represented by formula (1-1).

Further, use of relatively inexpensive bis(4-aminophenyl)amine as a raw material compound as described later allows relatively easy synthesis, and also due to excellent solubility in the organic solvent, it is preferred that the aniline derivative represented by formula (1) is the aniline derivative represented by formula (1-1).

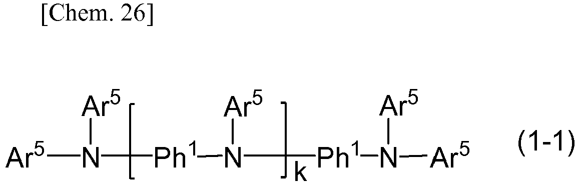

In the above formula (1-1), Ph¹ and k represent the same meaning as described above, Ar⁵ simultaneously represents the group represented by any one of formulae (D1) to (D13), and in particular, the group represented by any one of formulae (D1') to (D13') is preferred.

In addition, specific examples of Ar⁵ include those as described above as the specific examples of Ar¹. 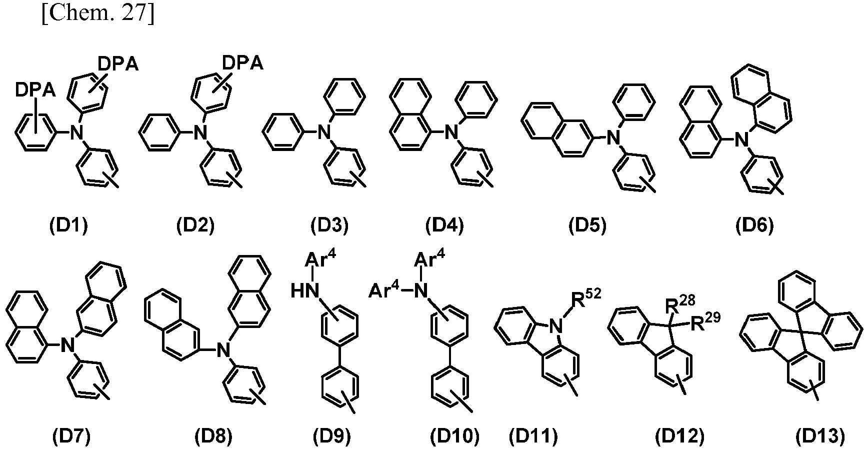 wherein R²⁸, R²⁹, R⁵², Ar⁴, and DPA represent the same meaning as described above. 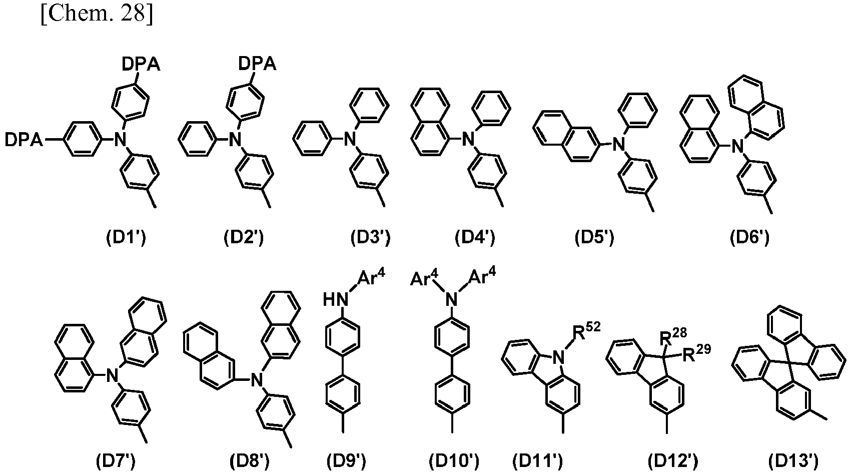 wherein R²⁸, R²⁹, R⁵², Ar⁴, and DPA represent the same meaning as described above.

Further, use of relatively inexpensive bis(4-aminophenyl)amine as a raw material compound as described later allows relatively easy synthesis, and due to excellent solubility of the resulting aniline derivative in the organic solvent, it is preferred that the aniline derivative represented by formula (1) is the aniline derivative represented by formula (1-2).

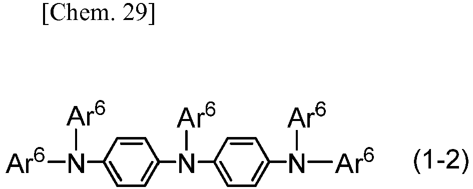

Ar⁶ simultaneously represents a group represented by any one of formulae (E1) to (E14). 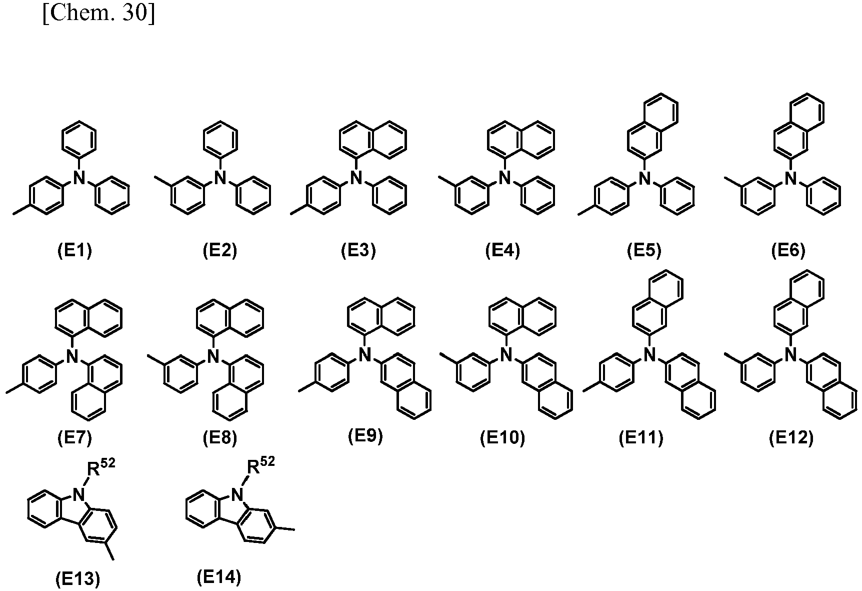 wherein R⁵² represents the same meaning as described above.

In the above formula (2), Ar³ represents a group represented by any one of formulae (C1) to (C8), and in particular, a group represented by any one of formulae (C1') to (C8') is preferred.

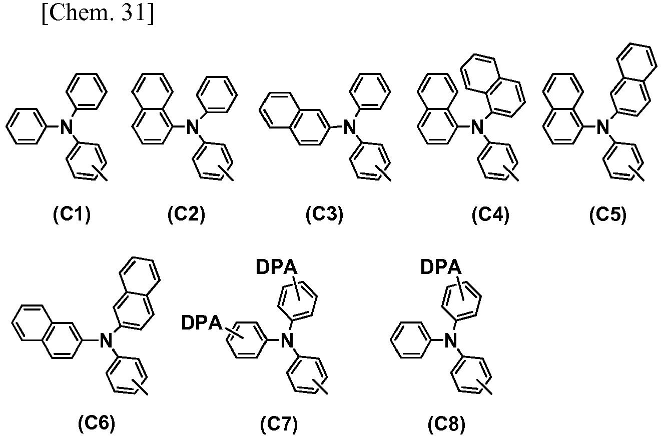

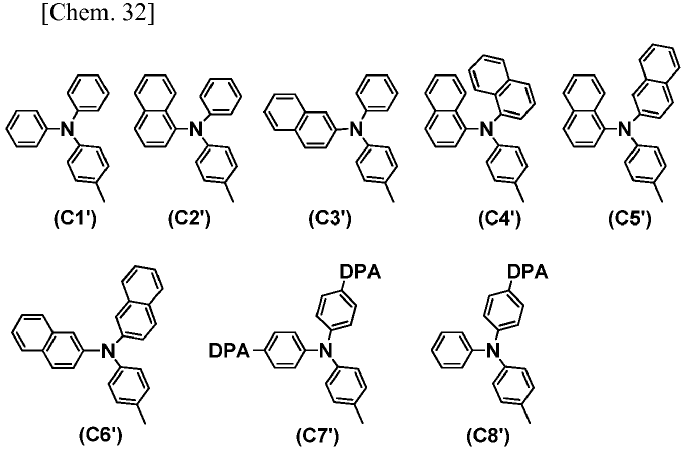

In the above formula (1), k represents an integer of 1 to 10, and from a viewpoint of raising solubility of the compound in an organic solvent, preferably 1 to 5, more preferably 1 to 3, still more preferably 1 or 2, and most preferably 1.

In the above formula (2), 1 represents 1 or 2.

In addition, in R²⁸, R²⁹, R⁵², and R¹⁵⁵ to R¹⁵⁷, Z¹ is preferably a halogen atom, a nitro group, a cyano group, an alkyl group having 1 to 10 carbon atoms which may be substituted by Z², an alkenyl group having 2 to 10 carbon atoms which may be substituted by Z², or an alkynyl group having 2 to 10 carbon atoms which may be substituted by Z², more preferably a halogen atom, a nitro group, a cyano group, an alkyl group having 1 to 3 carbon atoms which may be substituted by Z², an alkenyl group having 2 to 3 carbon atoms which may be substituted by Z², or an alkynyl having 2 to 3 carbon atoms which may be substituted by Z², and still more preferably a fluorine atom, an alkyl group having 1 to 3 carbon atoms which may be substituted by Z², an alkenyl group having 2 to 3 carbon atoms which may be substituted by Z², or an alkynyl group having 2 to 3 carbon atoms which may be substituted by Z².

In R²⁸, R²⁹, R⁵², and R¹⁵⁵ to R¹⁵⁷, Z⁴ is preferably a halogen atom, a nitro group, a cyano group, or an aryl group having 6 to 14 carbon atoms which may be substituted by Z⁵, more preferably a halogen atom, a nitro group, a cyano group, or an aryl group having 6 to 10 carbon atoms which may be substituted by Z⁵, still more preferably a fluorine atom or an aryl group having 6 to 10 carbon atoms which may be substituted by Z⁵, and further preferably a fluorine atom or a phenyl group which may be substituted by Z⁵.

In R²⁸, R²⁹, R⁵², and R¹⁵⁵ to R¹⁵⁷, Z² is preferably a halogen atom, a nitro group, a cyano group, or an aryl group having 6 to 14 carbon atoms which may be substituted by Z³, more preferably a halogen atom, a nitro group, a cyano group, or an aryl group having 6 to 10 carbon atoms which may be substituted by Z³, still more preferably a fluorine atom or an aryl group having 6 to 10 carbon atoms which may be substituted by Z³, and further preferably a fluorine atom or a phenyl group which may be substituted by Z³.

In R²⁸, R²⁹, R⁵², and R¹⁵⁵ to R¹⁵⁷, Z⁵ is preferably a halogen atom, a nitro group, a cyano group, an alkyl group having 1 to 10 carbon atoms which may be substituted by Z³, an alkenyl group having 2 to 10 carbon atoms which may be substituted by Z³, or an alkynyl group having 2 to 10 carbon atoms which may be substituted by Z³, more preferably a halogen atom, a nitro group, a cyano group, an alkyl group having 1 to 3 carbon atoms which may be substituted by Z³, an alkenyl group having 2 to 3 carbon atoms which may be substituted by Z³, or an alkynyl having 2 to 3 carbon atoms which may be substituted by Z³, and still more preferably a fluorine atom, an alkyl group having 1 to 3 carbon atoms which may be substituted by Z³, an alkenyl group having 2 to 3 carbon atoms which may be substituted by Z³, or an alkynyl group having 2 to 3 carbon atoms which may be substituted by Z³.

In R²⁸, R²⁹, R⁵², and R¹⁵⁵ to R¹⁵⁷, Z³ is preferably a halogen atom, and more preferably a fluorine atom.

Meanwhile, in R⁷ to R²⁷, R³⁰ to R⁵¹, and R⁵³ to R¹⁵⁴, Z¹ is preferably a halogen atom, a nitro group, a cyano group, an alkyl group having 1 to 3 carbon atoms which may be substituted by Z², an alkenyl group having 2 to 3 carbon atoms which may be substituted by Z², or an alkynyl group having 2 to 3 carbon atoms which may be substituted by Z², more preferably a halogen atom or an alkyl group having 1 to 3 carbon atoms which may be substituted by Z², and still more preferably a fluorine atom or a methyl group which may be substituted by Z².

In R⁷ to R²⁷, R³⁰ to R⁵¹, and R⁵³ to R¹⁵⁴, Z⁴ is preferably a halogen atom, a nitro group, a cyano group, or an aryl group having 6 to 10 carbon atoms which may be substituted by Z⁵, more preferably a halogen atom or an aryl group having 6 to 10 carbon atoms which may be substituted by Z⁵, and still more preferably a fluorine atom or a phenyl group which may be substituted by Z⁵.

In R⁷ to R²⁷, R³⁰ to R⁵¹, and R⁵³ to R¹⁵⁴, Z² is preferably a halogen atom, a nitro group, a cyano group, or an aryl group having 6 to 10 carbon atoms which may be substituted by Z³, more preferably a halogen atom or an aryl group having 6 to 10 carbon atoms which may be substituted by Z³, and still more preferably a fluorine atom or a phenyl group which may be substituted by Z³.

In R⁷ to R²⁷, R³⁰ to R⁵¹, and R⁵³ to R¹⁵⁴, Z⁵ is preferably a halogen atom, a nitro group, a cyano group, an alkyl group having 1 to 3 carbon atoms which may be substituted by Z³, an alkenyl group having 2 to 3 carbon atoms which may be substituted by Z³, or an alkynyl group having 2 to 3 carbon atoms which may be substituted by Z³, more preferably a halogen atom or an alkyl group having 1 to 3 carbon atoms which may be substituted by Z³, and still more preferably a fluorine atom or a methyl group which may be substituted by Z³.

In R⁷ to R²⁷, R³⁰ to R⁵¹, and R⁵³ to R¹⁵⁴, Z³ is preferably a halogen atom, and more preferably a fluorine atom.

Specific examples of the group suitable as R⁵² and R¹⁵⁵ include the following groups, but are not necessarily limited thereto.

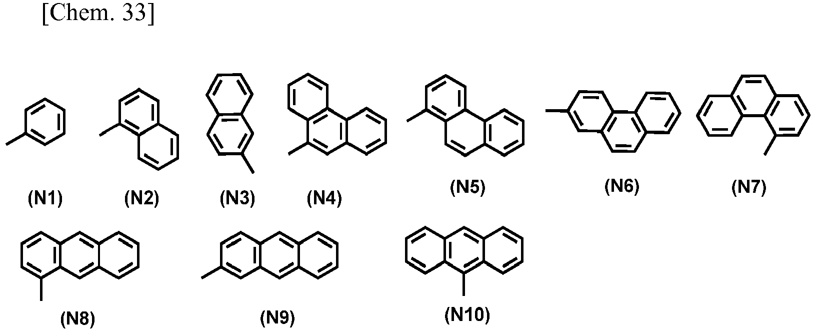

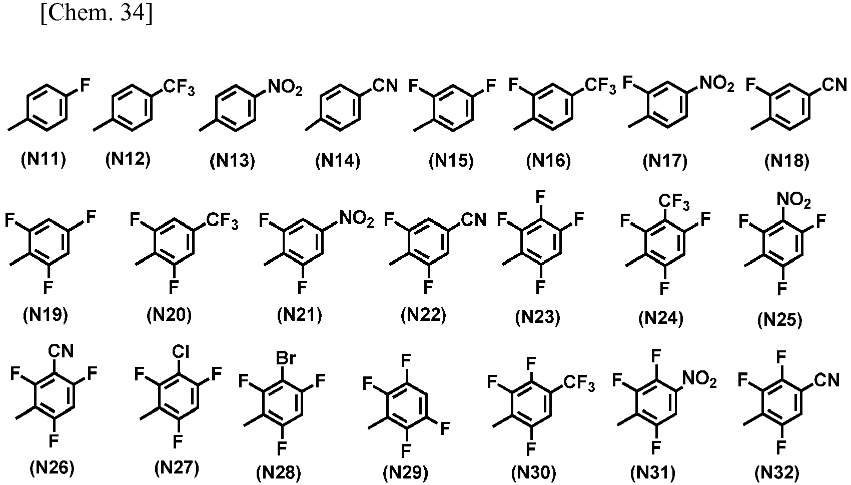

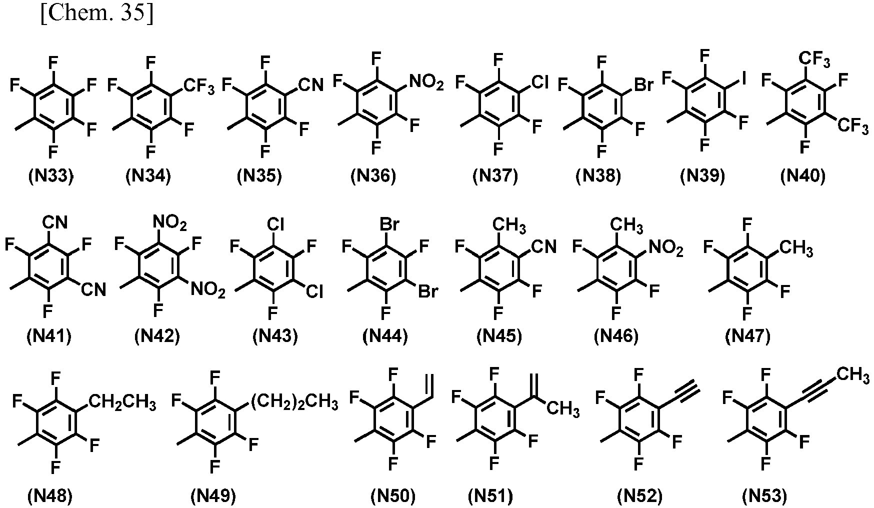

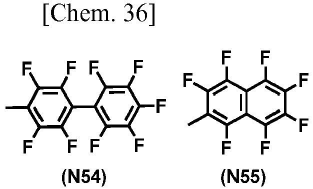

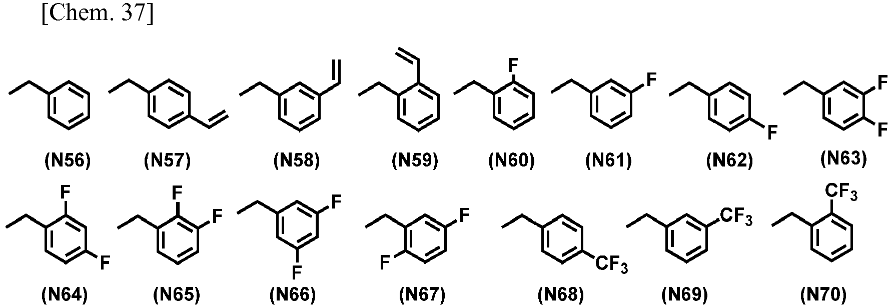

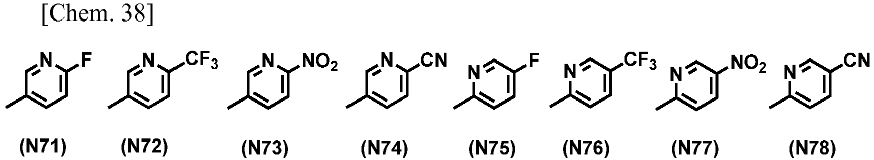

The numbers of carbon atoms of the alkyl group, the alkenyl group, and the alkynyl group are preferably 10 or less, more preferably 6 or less, and still more preferably 4 or less.

Further, the numbers of carbon atoms of the aryl group and the heteroaryl group are preferably 14 or less, more preferably 10 or less, and still more preferably 6 or less.

The aniline derivative represented by the above formula (1) can be produced by reacting the amine compound represented by formula (3) with the aryl compound represented by formula (4) in the presence of a catalyst. 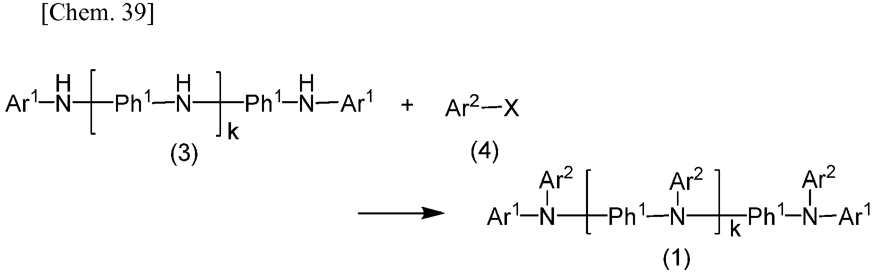 wherein X represents a halogen atom or a pseudo halogen group, and Ar¹, Ar², Ph¹, and k represent the same meaning as described above.

In particular, the aniline derivative represented by formula (1-1) can be produced by reacting the amine compound represented by formula (5) with the aryl compound represented by formula (6) in the presence of a catalyst. 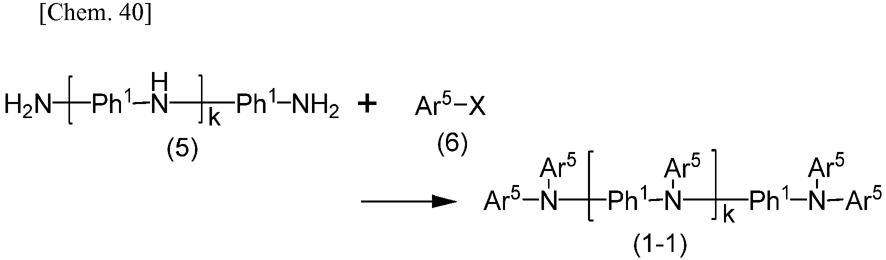 wherein X, Ar⁵, Ph¹, and k represent the same meaning as described above.

Further, the aniline derivative represented by the above formula (1-2) can be produced by reacting bis(4-aminophenyl)amine with the aryl compound represented by formula (7) in the presence of a catalyst. 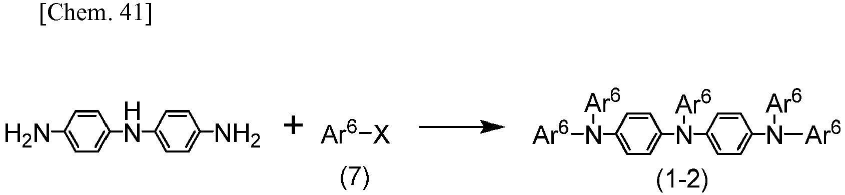 wherein X and Ar⁶ represent the same meaning as described above.

Meanwhile, the aniline derivative represented by the above formula (2) can be produced by reacting the amine compound represented by formula (8) with the aryl compound represented by formula (9) in the presence of a catalyst. 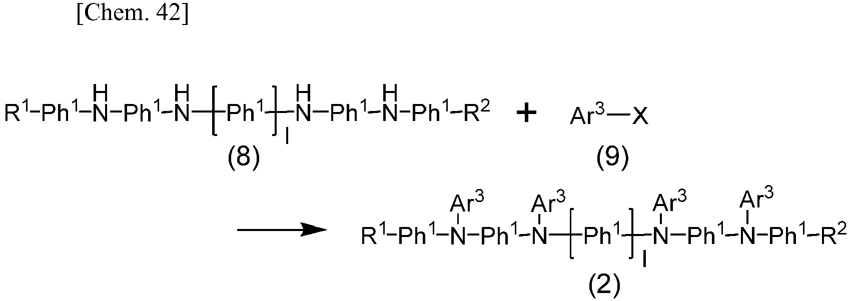 wherein X, R¹, R², Ar³, Ph¹, and 1 represent the same meaning as described above.

Examples of the halogen atom include those as described above.

Examples of the pseudo halogen atom include (fluoro)alkylsulfonyloxy groups such as a methanesulfonyloxy group, a trifluoromethanesulfonyloxy group, and a nonafluorobutanesulfonyloxy group; aromatic sulfonyloxy groups such as a benzenesulfonyloxy group and a toluenesulfonyloxy group; and the like.

A charge ratio between the amine compound represented by formula (3), (5), or (8), or bis(4-aminophenyl)amine and the aryl compound represented by formula (4), (6), (7), or (9) can be equal to or more than the equivalent of the aryl compound, and preferably about 1 to 1.2 equivalents of the aryl compound, per a substance amount of total NH groups of the amine compound or bis(4-aminophenyl)amine.

Examples of the catalyst used in the reaction include copper catalysts such as copper chloride, copper bromide, and copper iodide; palladium catalysts such as Pd(PPh₃)₄(tetrakis(triphenylphosphine)palladium), Pd(PPh₃)₂Cl₂(bis(triphenylphosphine)dichloropalladium), Pd(dba)₂(bis(dibenzylideneacetone)palladium), Pd₂(dba)₃(tris(dibenzylideneacetone)dipalladium), Pd(P-t-Bu₃)₂(bis(tri(t-butylphosphine))palladium), and Pd(OAc)₂(palladium acetate); and the like. These catalysts may be used alone or in combination of two or more. Further, these catalysts may be used with a known appropriate ligand.

Examples of such a ligand include tertiary phosphines such as triphenylphosphine, tri-o-tolylphosphine, diphenylmethylphosphine, phenyldimethylphosphine, trimethylphosphine, triethylphosphine, tributylphosphine, tri-tert-butylphosphine, di-t-butyl(phenyl)phosphine, di-tert-butyl(4-dimethylaminophenyl)phosphine, 1,2-bis(diphenylphosphino)ethane, 1,3 -bis(diphenylphosphino)propane, 1,4-bis(diphenylphosphino)butane, and 1,1'-bis(diphenylphosphino)ferrocene, tertiary phosphites such as trimethylphosphite, triethylphosphite, and triphenylphosphite, and the like.

A use amount of the catalyst can be about 0.2 mol, and preferably 0.15 mol per 1 mol of the aryl compound represented by formula (4), (6), (7), or (9).

Further, if the ligand is used, the use amount thereof can be 0.1 to 5 equivalents, and preferably 1 to 2 equivalents, per the metal complex to be used.

If the raw material compound is all solid, or from a viewpoint of efficiently obtaining the intended aniline derivative, each of the above reactions is carried out in a solvent. If the solvent is used, the type thereof is not particularly limited as long as it does not adversely affect the reaction. Specific examples thereof include aliphatic hydrocarbons (such as pentane, n-hexane, n-octane, n-decane, and decaline), halogenated aliphatic hydrocarbons (such as chloroform, dichloromethane, dichloroethane, and carbon tetrachloride), aromatic hydrocarbons (such as benzene, nitrobenzene, toluene, o-xylene, m-xylene, p-xylene, and mesitylene), halogenated aromatic hydrocarbons (such as chlorobenzene, bromobenzene, o-dichlorobenzene, m-dichlorobenzene, and p-dichlorobenzene), ethers (such as diethyl ether, diisopropyl ether, t-butylmethyl ether, tetrahydrofuran, dioxane, 1,2-dimethoxyethane, and 1,2-diethoxyethane), ketones (such as acetone, methylethylketone, methylisobutylketone, di-n-butylketone, and cyclohexanone), amides (such as N,N-dimethylformamide and N,N-dimethylacetamide), lactams and lactones (such as N-methylpyrrolidone and γ-butyrolactone), ureas (such as N,N-dimethylimidazolidinone and tetramethylurea), sulfoxides (such as dimethylsulfoxide and sulfolane), nitriles (such as acetonitrile, propionitrile, and butyronitrile), and the like, and these solvents may be used alone or in combination of two or more.

A reaction temperature may be appropriately set in a range from the melting point to the boiling point of the solvent to be used, but in particular, the reaction temperature is preferably about 0 to 200°C and more preferably 20 to 150°C.

After completion of the reaction, work-up is carried out by a usual method and the intended aniline derivative can be obtained.

In a method of producing the aniline derivative represented by the above formula (1), the amine compound represented by formula (3') used as a raw material can be efficiently produced by reacting the amine compound represented by formula (10) with the aryl compound represented by formula (11) in the presence of a catalyst. 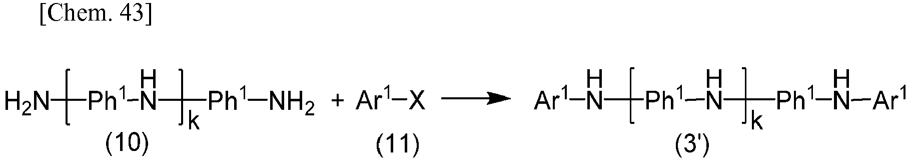 wherein X, Ar¹, Ph¹, and k represent the same meaning as described above, provided that two Ar¹'s are not the group represented by formula (B1) at the same time.

In the method of producing the amine compound represented by formula (3'), the amine compound represented by formula (10) and the aryl compound represented by formula (11) are subjected to a coupling reaction, and it is preferred that a charge ratio between the amine compound represented by formula (10) and the aryl compound represented by formula (11) is about 2 to 2.4 of the aryl compound per 1 of the amine compound, as a substance amount ratio.

In addition, the conditions for the catalyst, the ligand, the solvent, the reaction temperature, and the like in the coupling reaction are the same as the conditions described above for the method of producing the aniline derivative represented by formula (1).

In formula (1), when producing the aniline derivative wherein Ar¹ is the group represented by formula (B4) in which R⁵² is a hydrogen atom or the group represented by formula (B10), or Ar² is the group represented by formula (A12) or the group represented by formula (A16) in which R¹⁵⁵ (in formula (1-1), R⁵² is included) is a hydrogen atom, an aryl compound having a known protecting group on an amino group may be used, in the above-described reaction.

Hereinafter, specific examples of the aniline derivative represented by formula (1) or (2) are shown, but are not limited thereto. In the formulae and tables, "Me" represents a methyl group, "Et" represents an ethyl group, "Prⁿ" represents an n-propyl group, "Prⁱ" represents an i-propyl group, "Buⁿ" represents an n-butyl group, "Buⁱ" represents an i-butyl group, "Bu^{s}" represents a s-butyl group, "Bu^{t}" represents a t-butyl group, "DPA" represents a diphenylamino group, and "SBF" represents a 9,9'-spyrobi[9H-fluorene]-2-yl group.

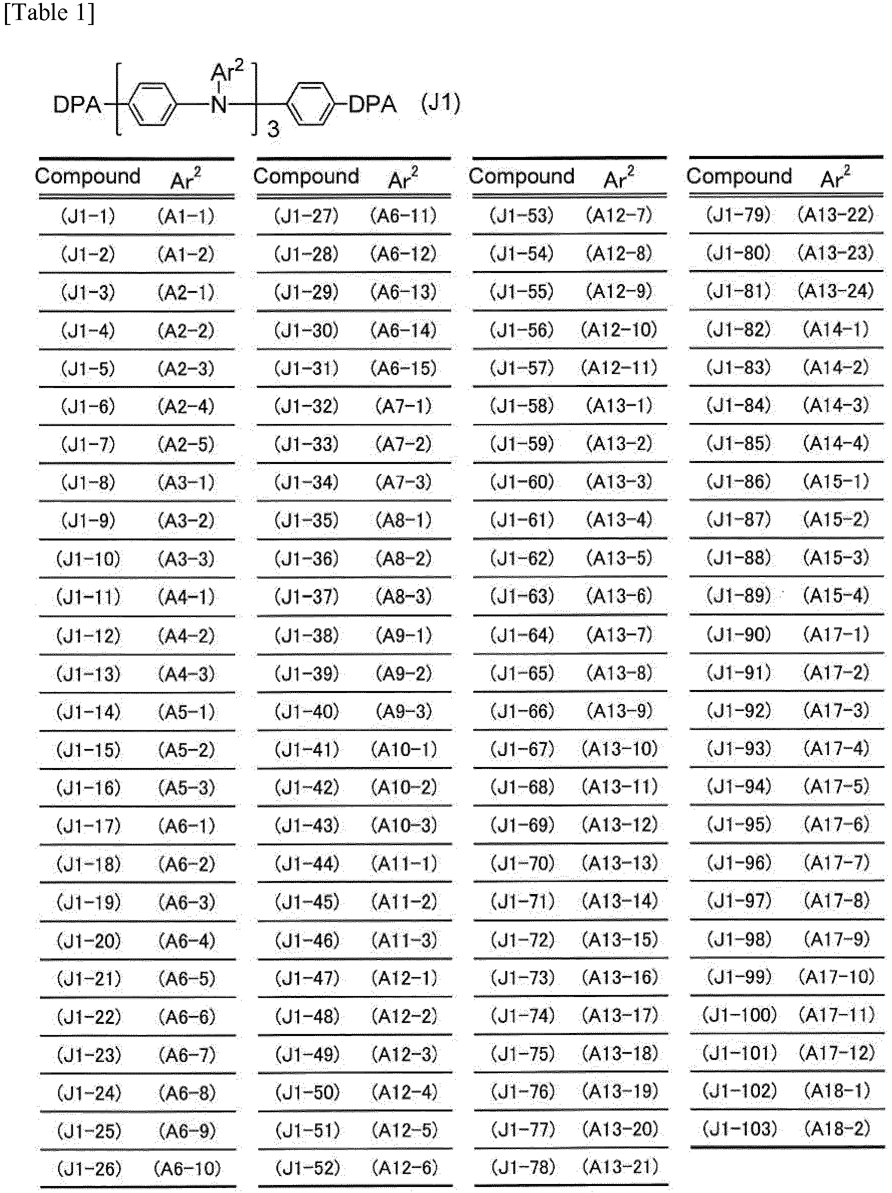

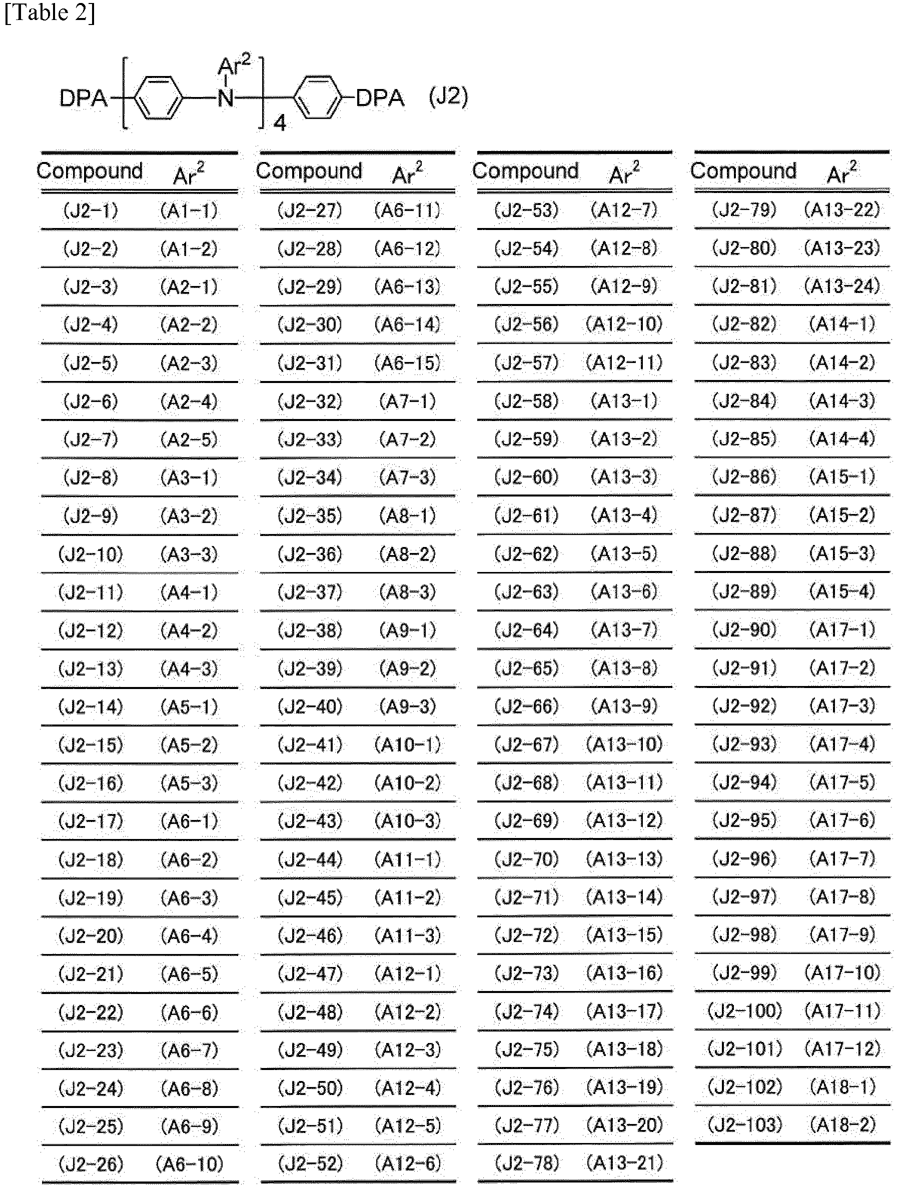

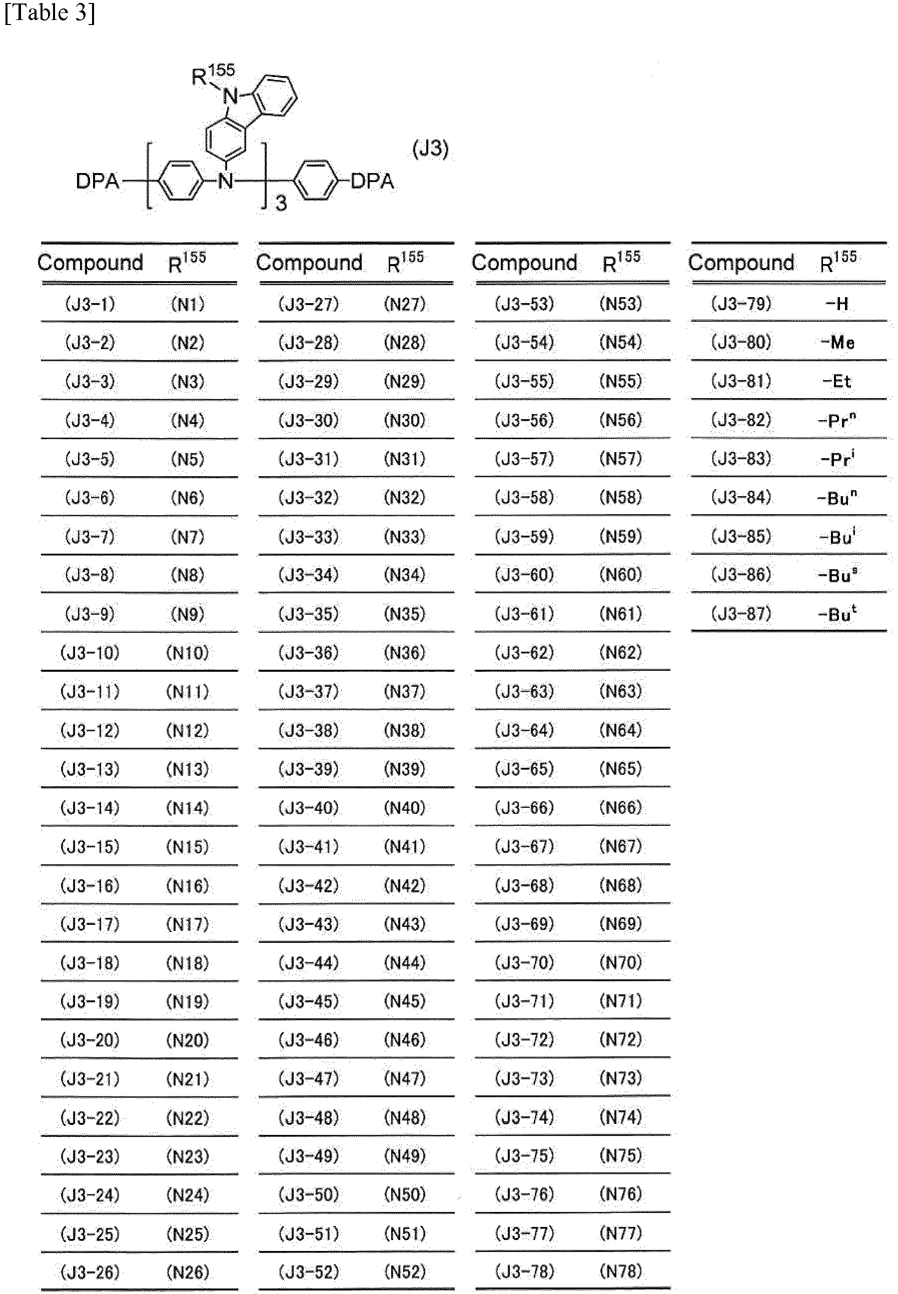

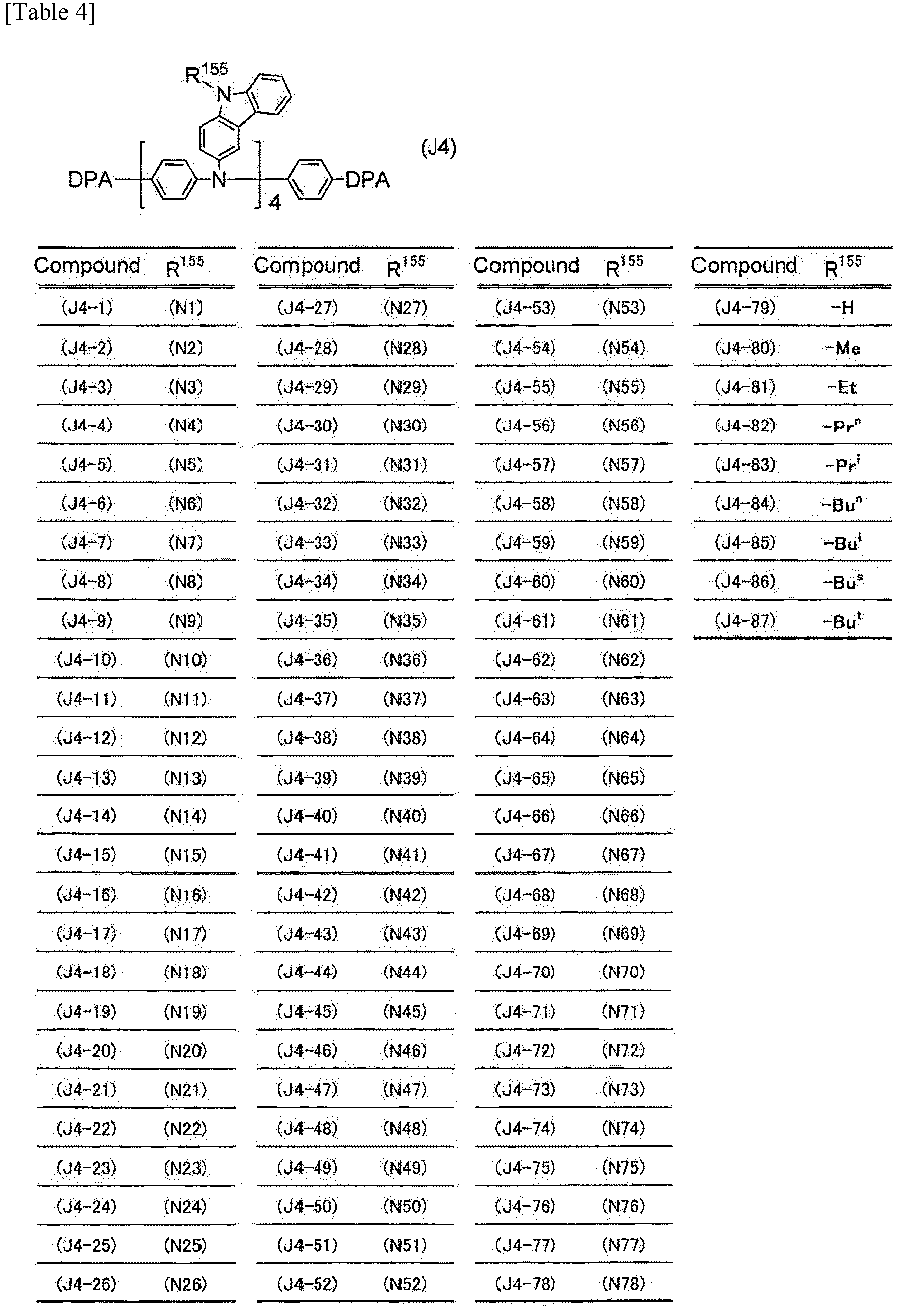

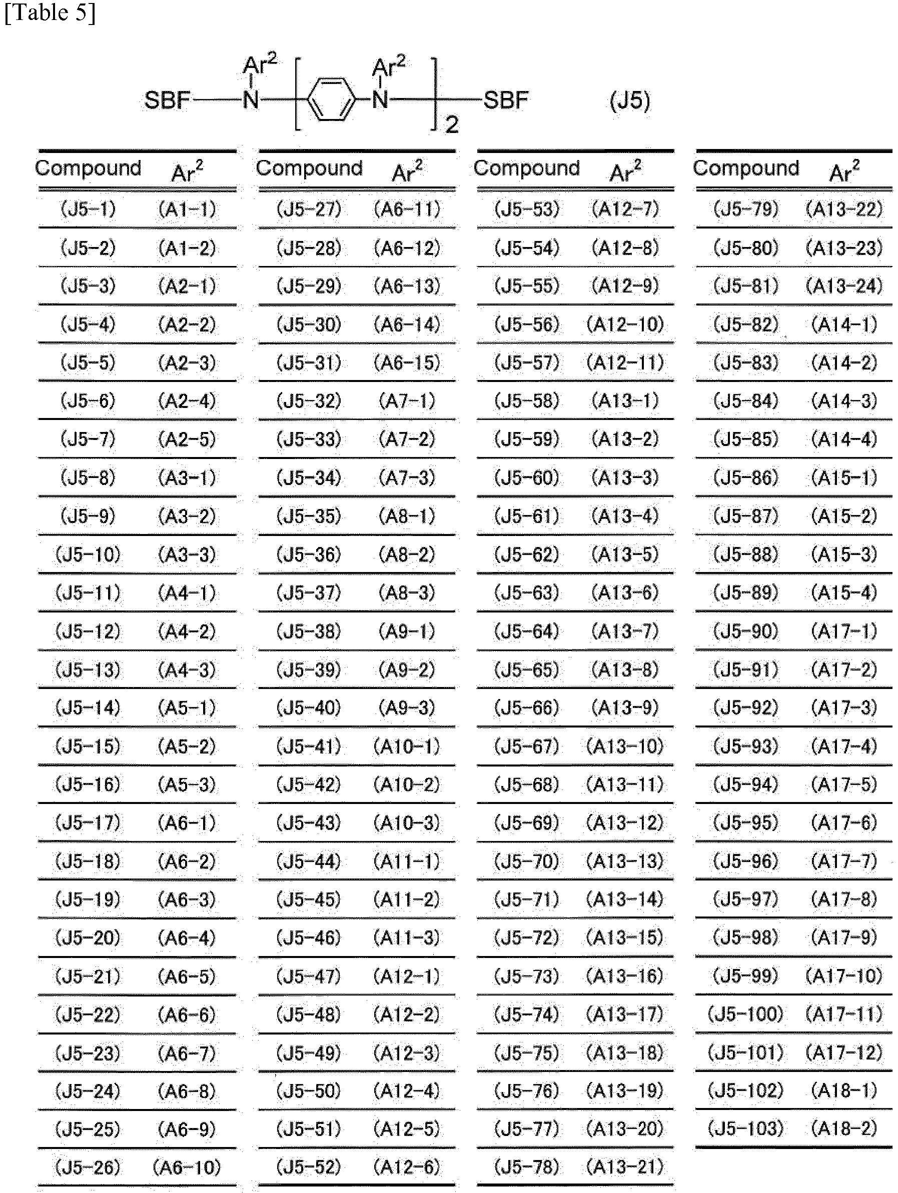

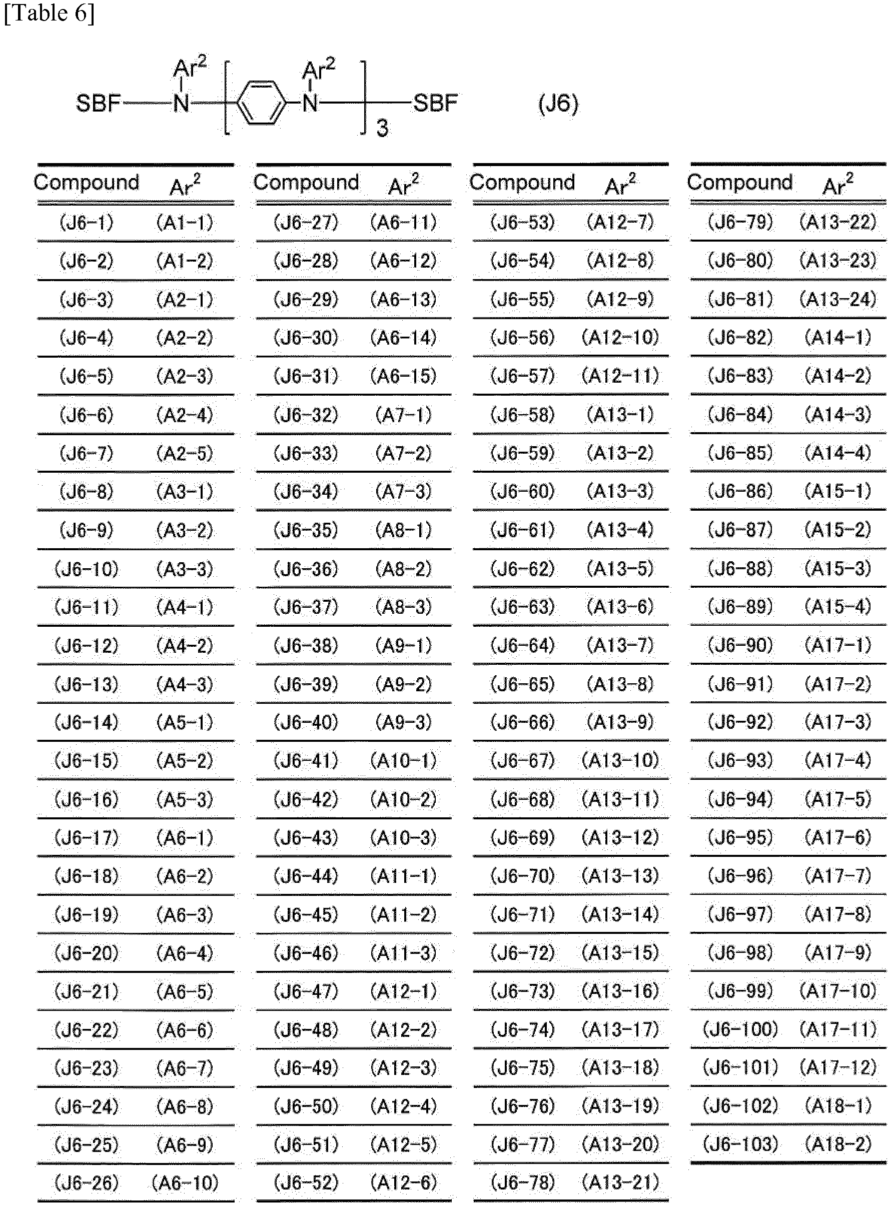

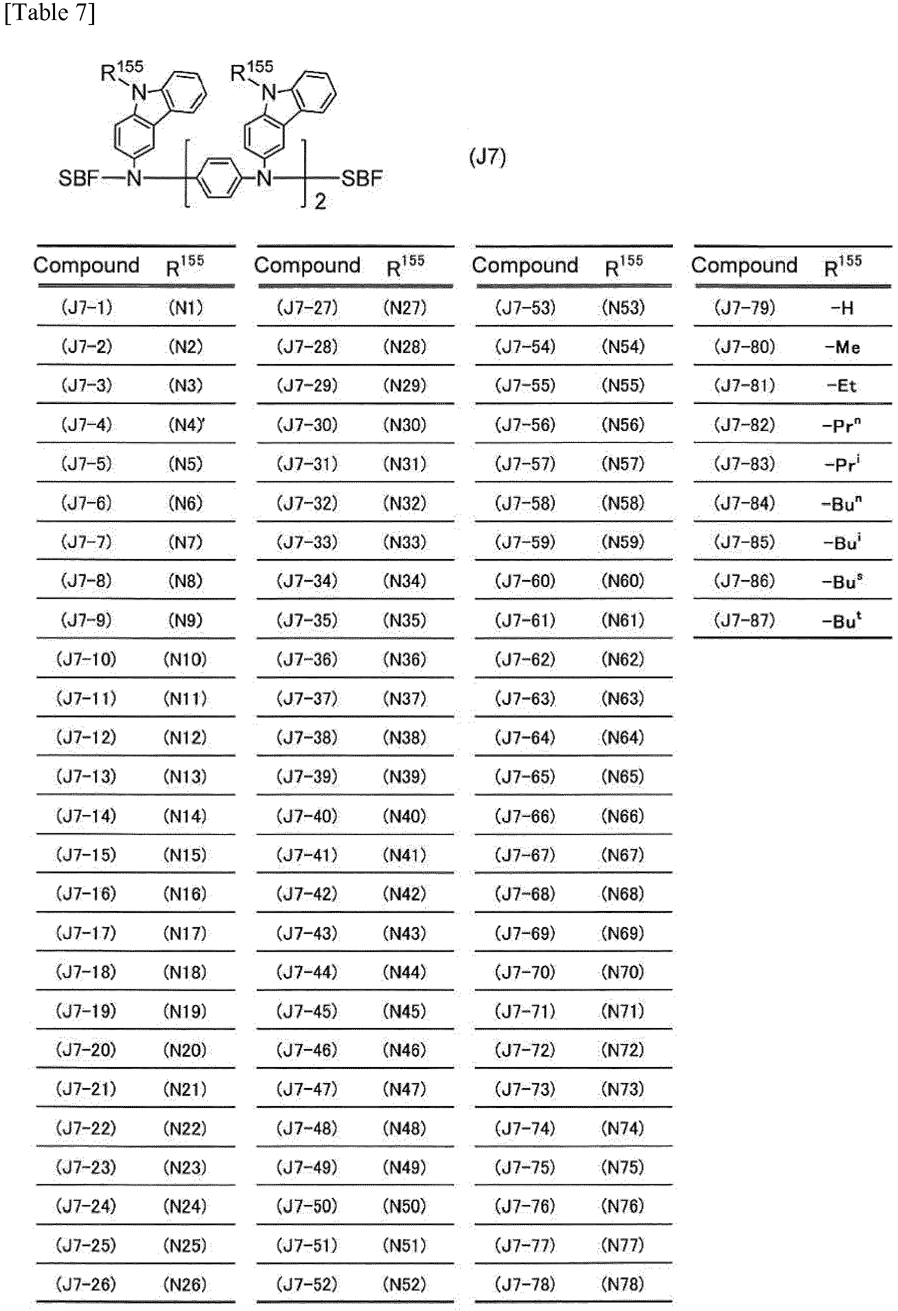

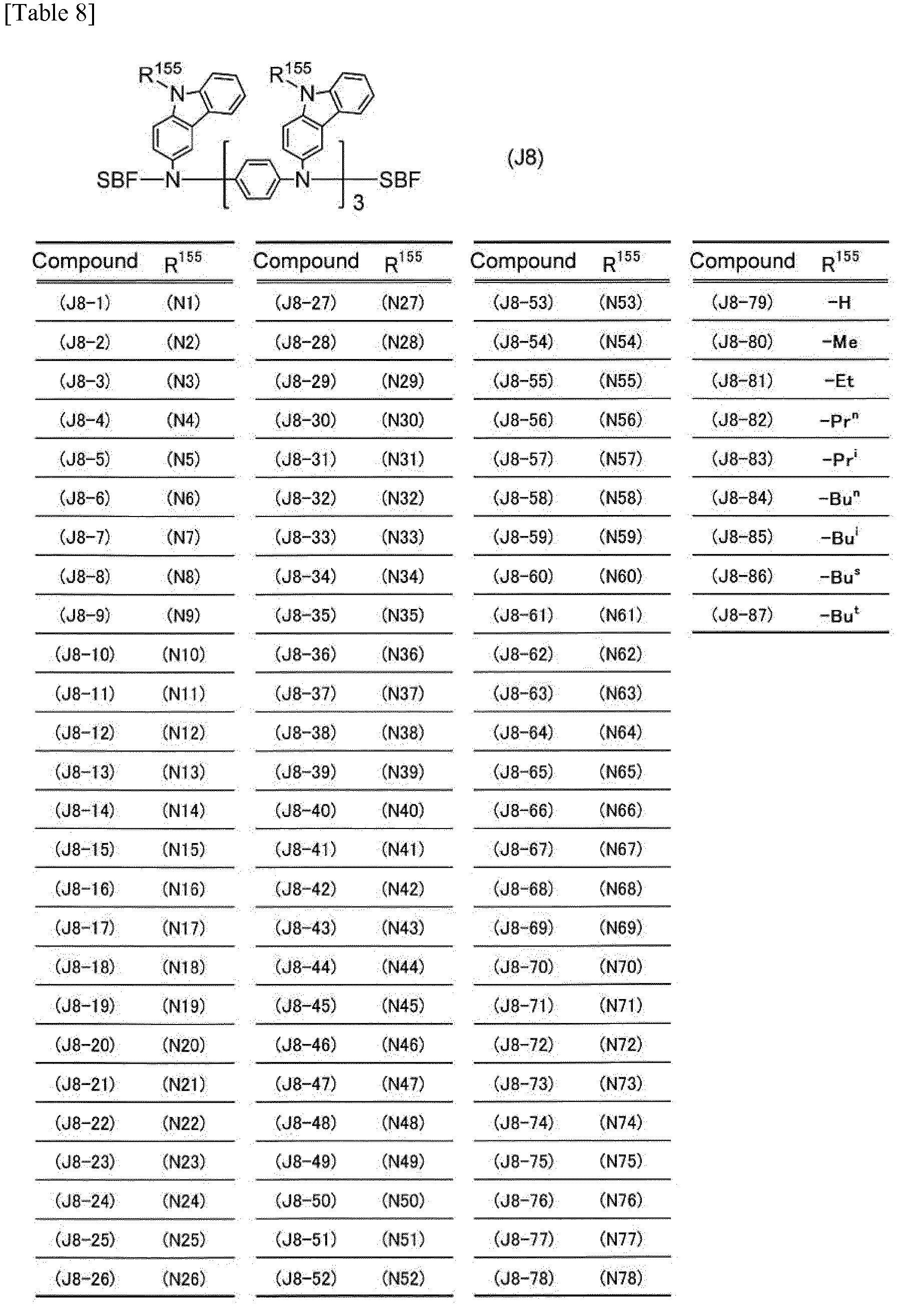

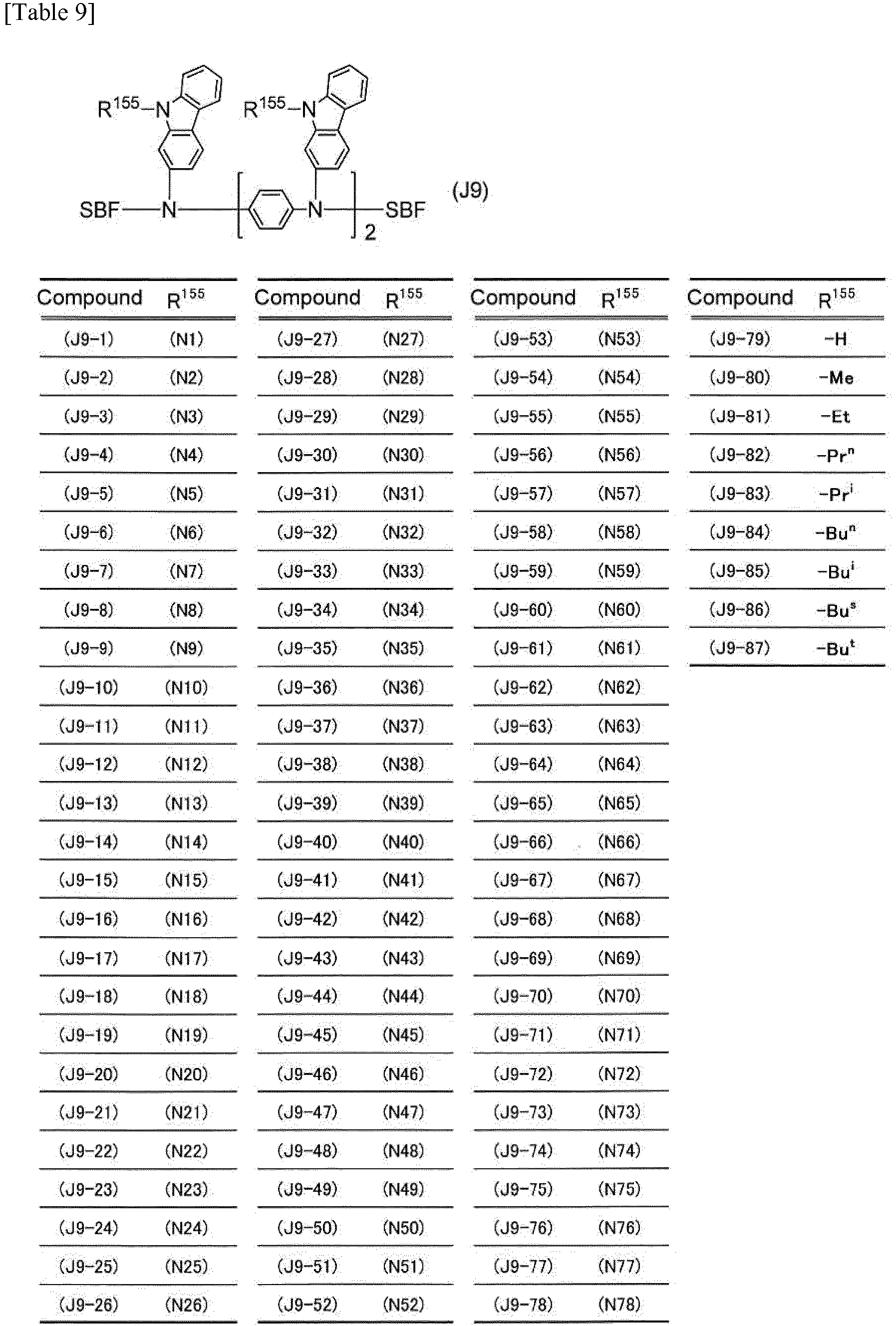

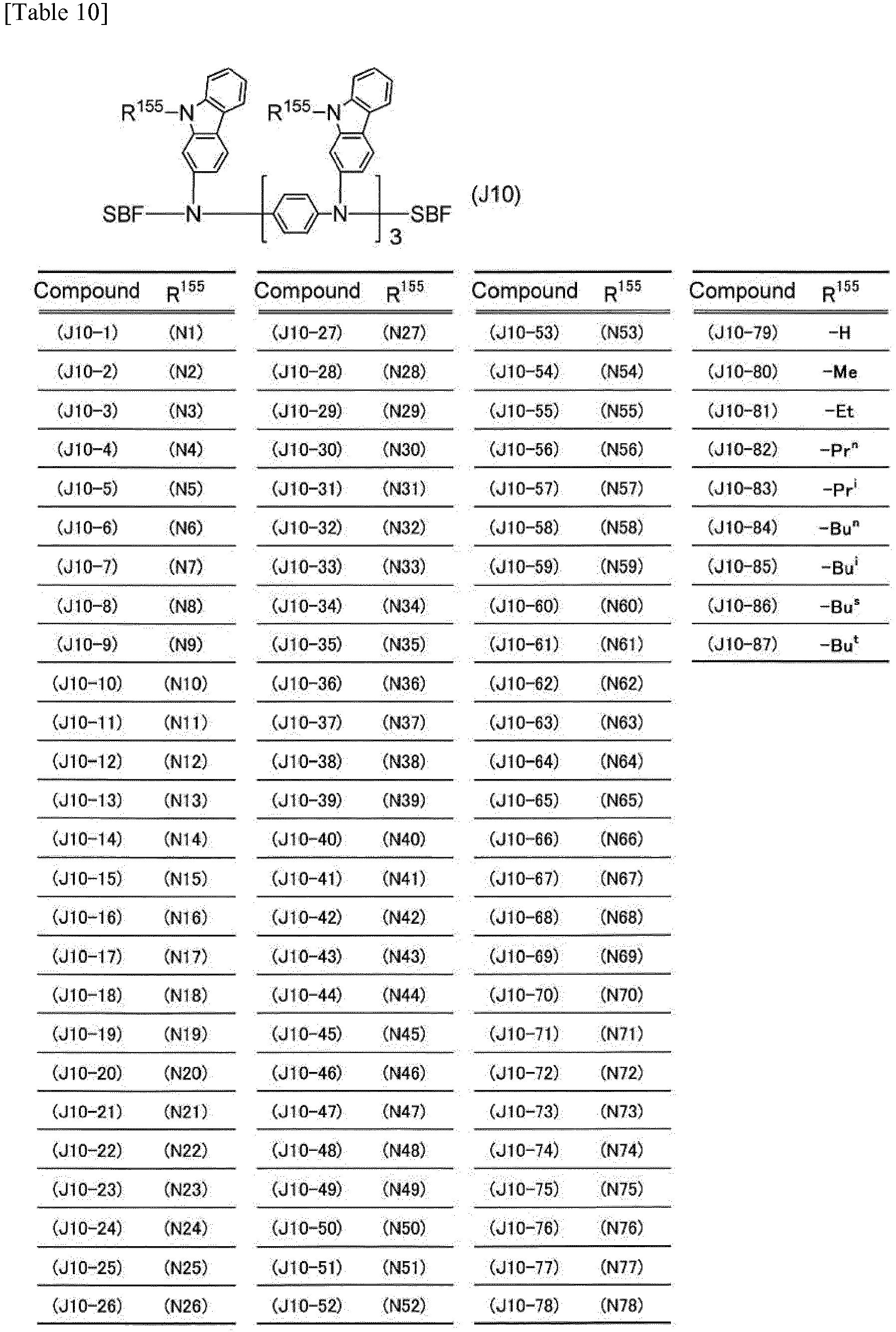

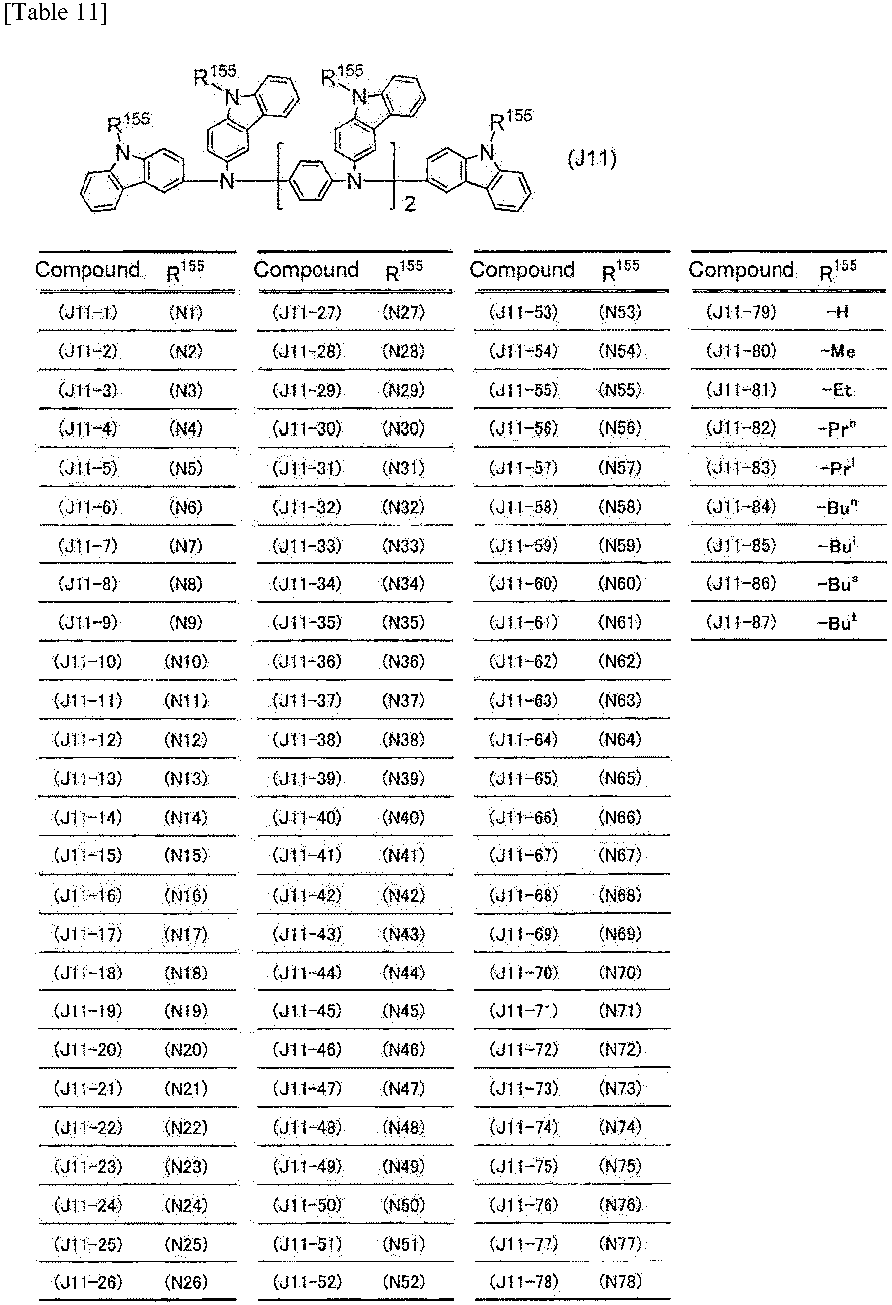

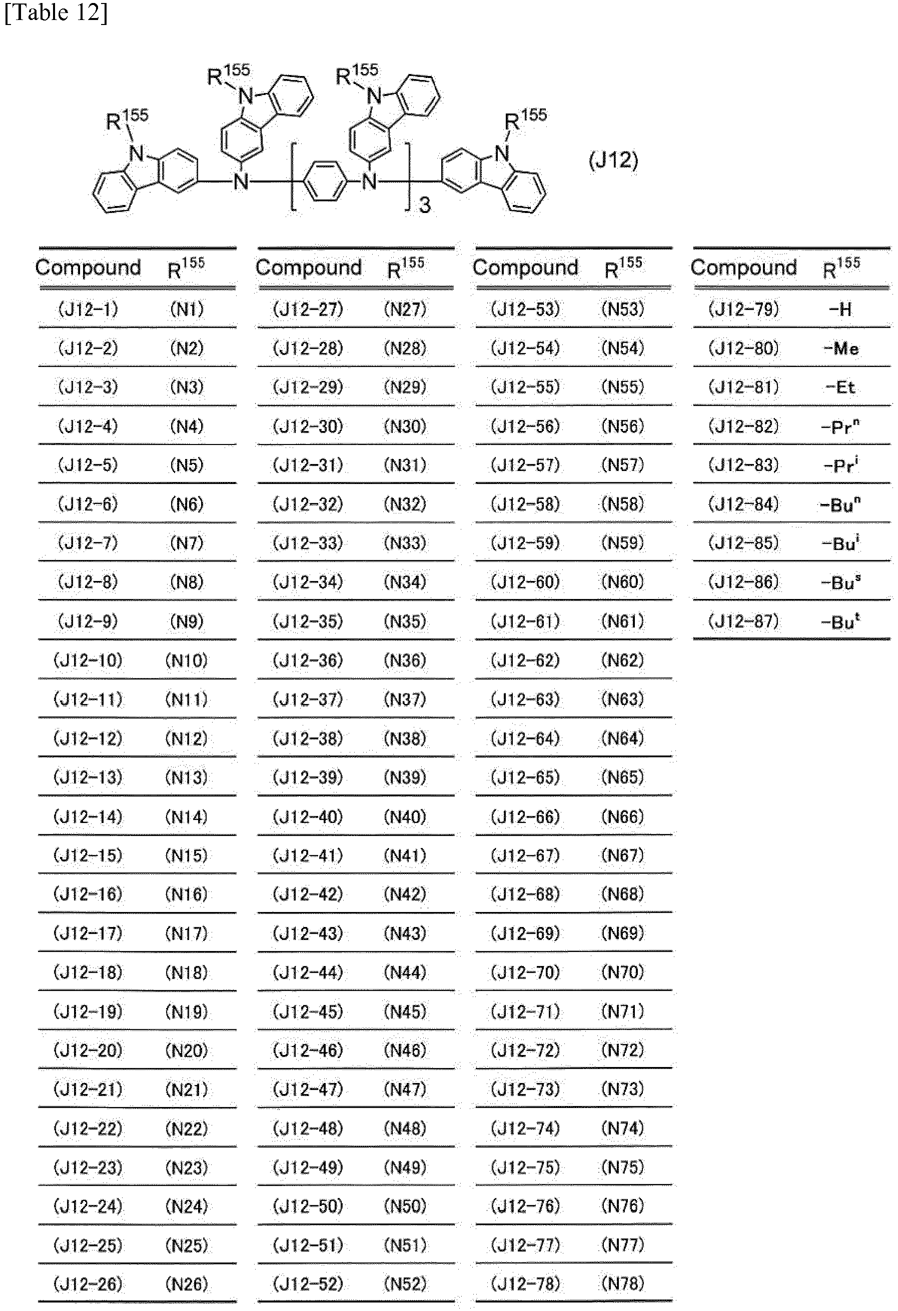

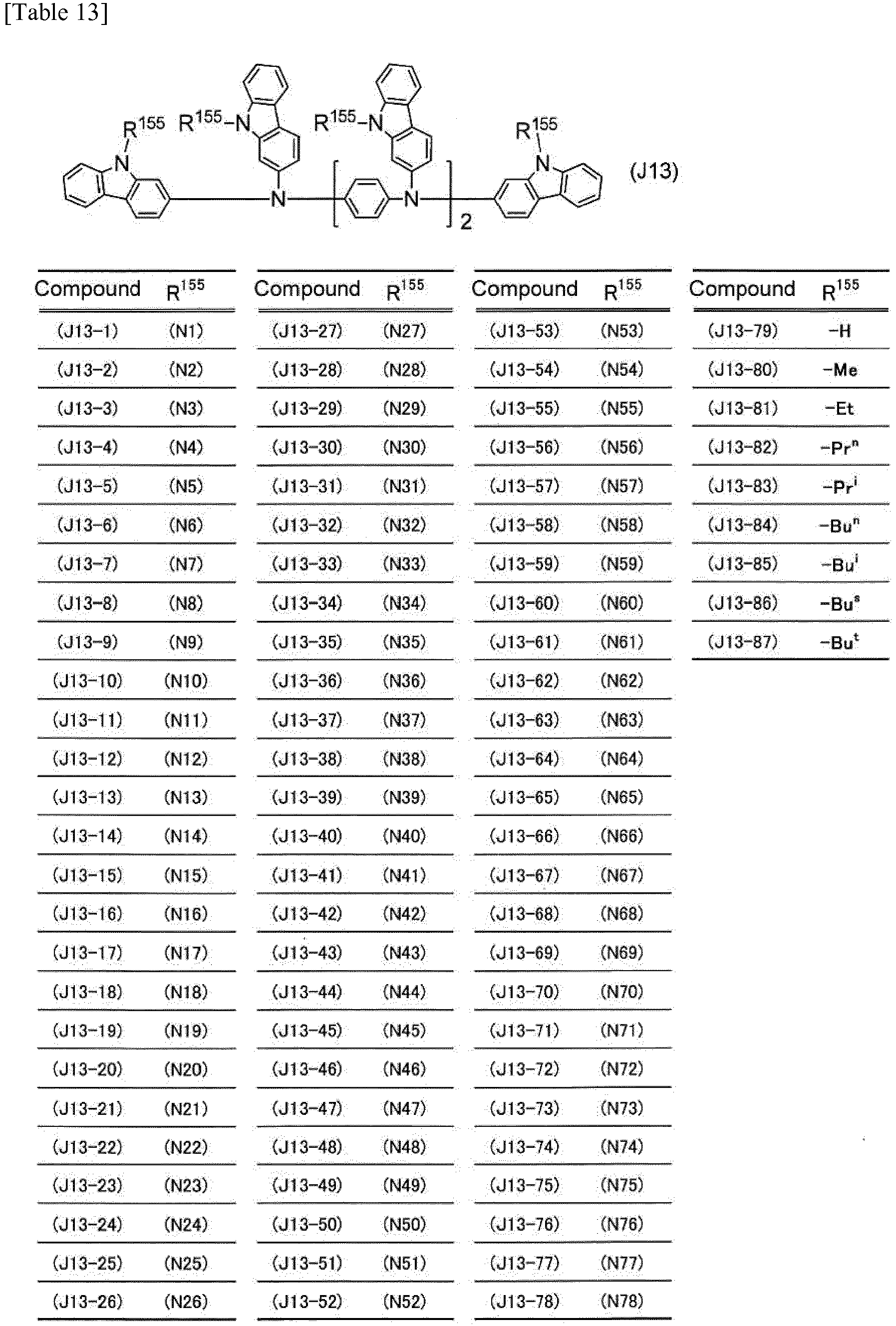

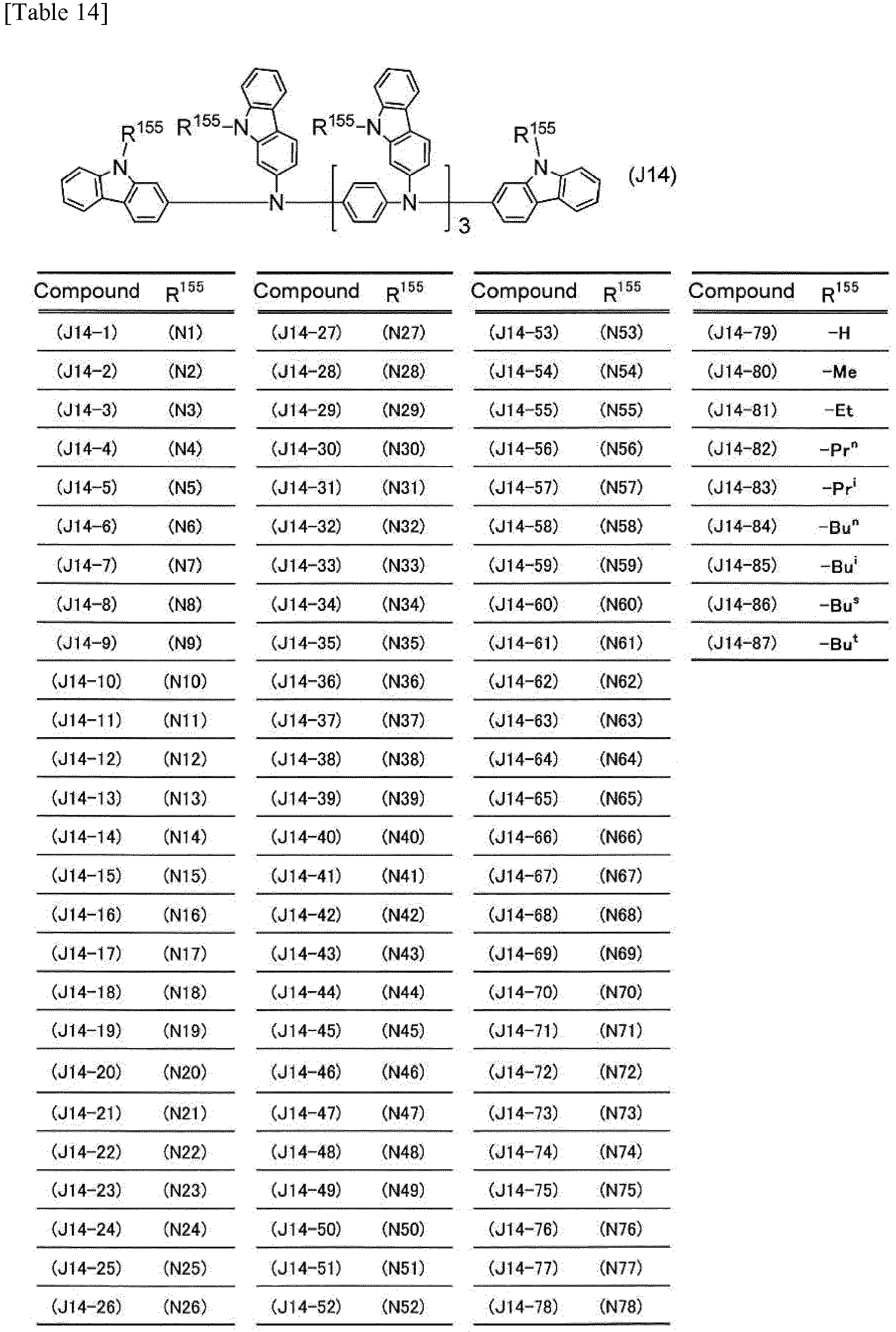

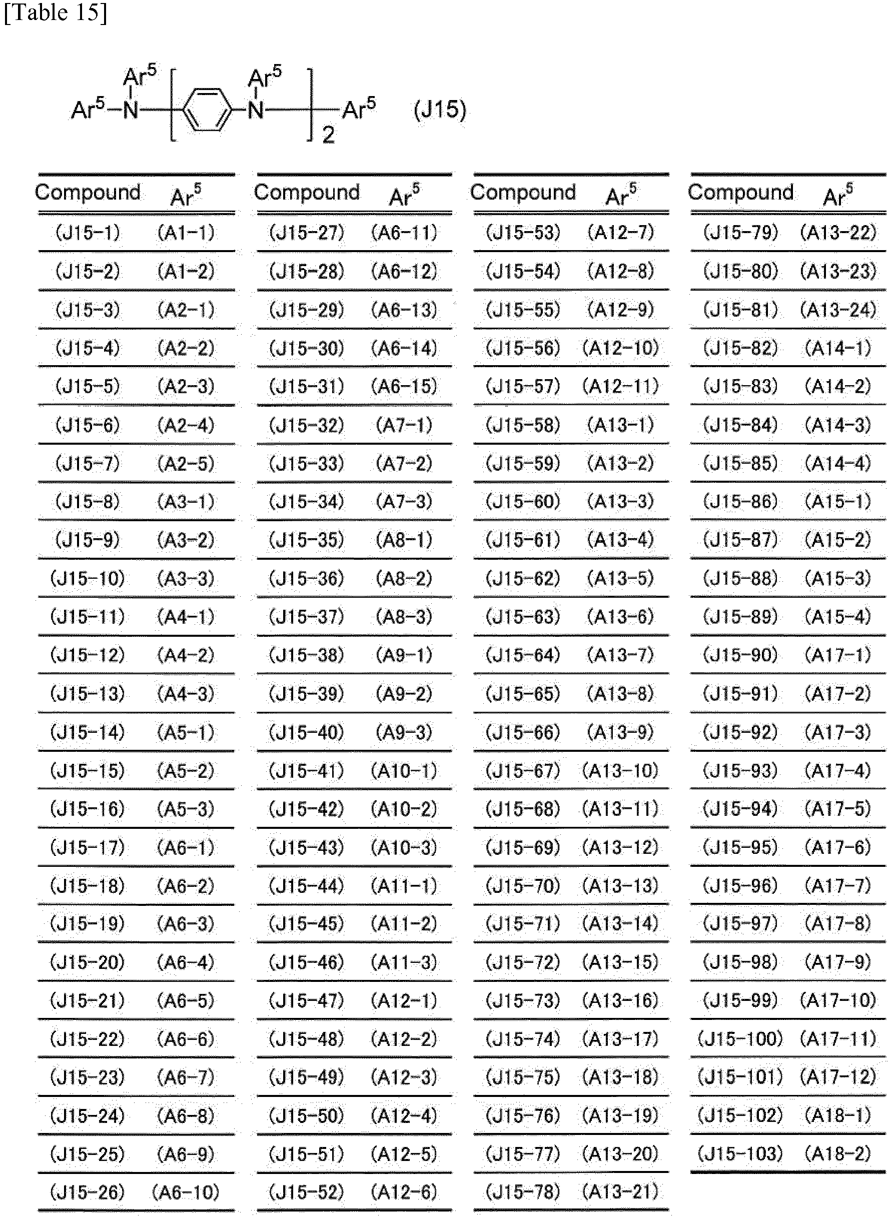

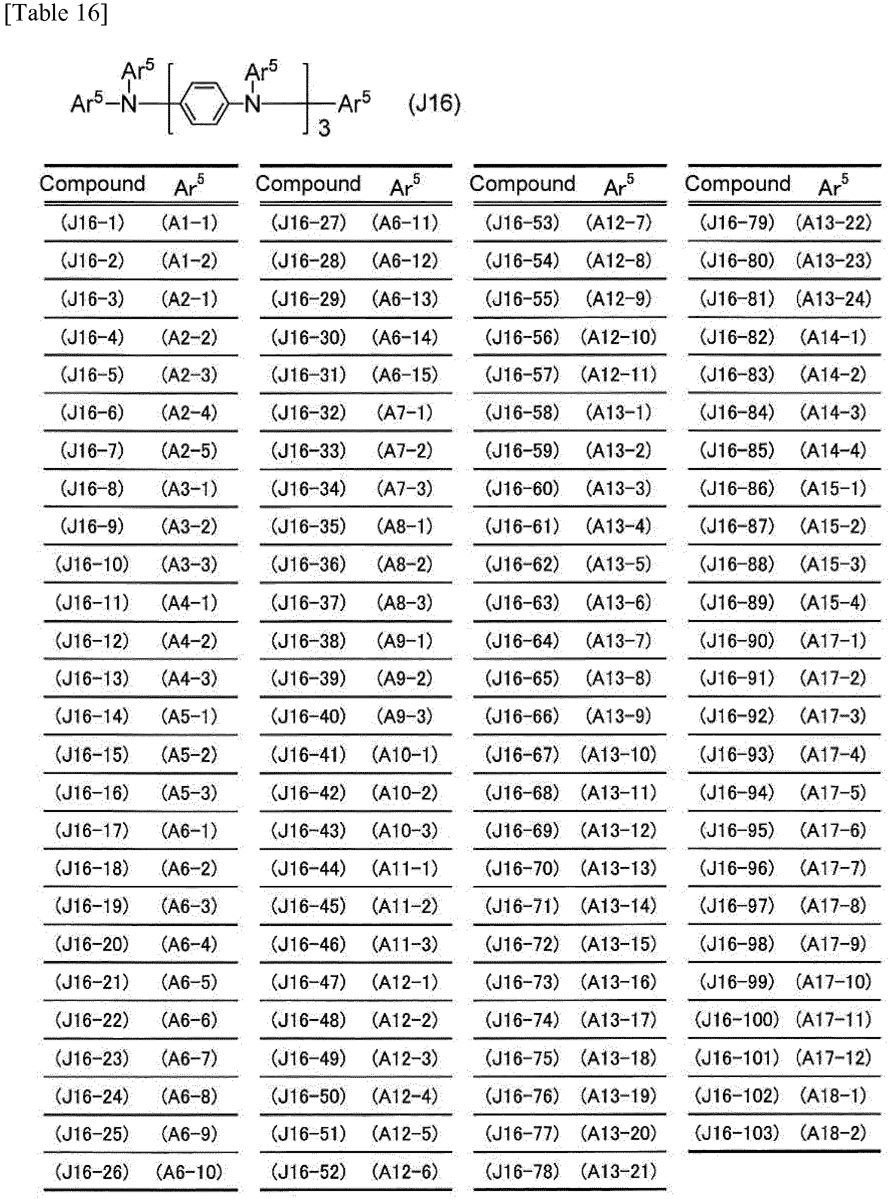

**[Table 17]**

| | | | | | | | |
|---|---|---|---|---|---|---|---|
| 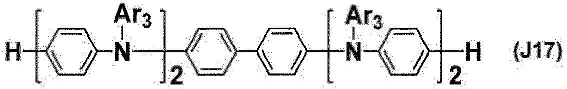 | | | | | | | |

| Compound | Ar³ | Compound | Ar³ | Compound | Ar³ | Compound | Ar³ |
|---|---|---|---|---|---|---|---|
| (J17-1) | (C1') | (J17-3) | (C3') | (J17-5) | (C5') | (J17-7) | (C7') |
| (J17-2) | (C2') | (J17-4) | (C4') | (J17-6) | (C6') | (J17-8) | (C8') |

**[Table 18]**

| | | | | | | | |
|---|---|---|---|---|---|---|---|
| 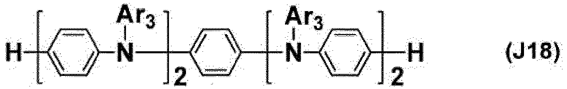 | | | | | | | |

| Compound | Ar³ | Compound | Ar³ | Compound | Ar³ | Compound | Ar³ |
|---|---|---|---|---|---|---|---|
| (J18-1) | (C1') | (J18-3) | (C3') | (J18-5) | (C5') | (J18-7) | (C7') |
| (J18-2) | (C2') | (J18-4) | (C4') | (J18-6) | (C6') | (J18-8) | (C8') |

In the present invention, a ratio between the fluorinated alkyl phosphate onium salt and the charge-transporting material can be charge-transporting material: fluorinated alkyl phosphate onium salt = about 1 : 0.1 to 10, in a substance amount (mole) ratio.

As the organic solvent used in preparing the charge-transporting varnish, a highly soluble solvent capable of dissolving the charge-transporting material and the fluorinated alkyl phosphate onium salt can be used.

Examples of such a highly soluble solvent include organic solvents such as cyclohexanone, N,N-dimethylformamide, N,N-dimethylacetamide, N-methyl pyrrolidone, 1,3-dimethyl-2-imidazolidinone, diethylene glycol monomethyl ether, 3-phenoxytoluene, 4-methoxytoluene, toluene, anisole, cyclohexylbenzene, methyl benzoate, tetralin, and isophorone, but are not limited thereto. These solvents can be used alone or in combination of two or more, and the use amount thereof can be 5 to 100% by weight per the entire solvent used in the varnish.

Further, in the present invention, the varnish includes at least one high-viscosity solvent having a viscosity of 10 to 200 mPa·s, particularly 35 to 150 mPa·s at 25°C and a boiling point of 50 to 300°C, particularly 150 to 250°C under normal pressure (atmospheric pressure), whereby it is easy to adjust the viscosity of the varnish, and as a result, it is possible to prepare a varnish according to an application method, which provides a thin film having high flatness with good producibility.

Examples of the high-viscosity organic solvent include cyclohexanol, ethylene glycol, ethylene glycol diglycidyl ether, 1,3-octylene glycol, diethylene glycol, dipropylene glycol, triethylene glycol, tripropylene glycol, 1,3-butanediol, 2,3-butanediol, 1,4-butanediol, propylene glycol, hexylene glycol, and the like, but are not limited thereto. These solvents may be used alone or in combination of two or more.

The addition ratio of the high-viscosity organic solvent to the entire solvent used in the varnish of the present invention is preferably in a range in which solids are not precipitated, and the addition ratio in the range in which solids are not precipitated is preferably 5 to 90% by weight.

In addition, for the purpose of improving wettability to a substrate, adjusting surface tension of the solvent, adjusting polarity, adjusting a boiling point, and the like, other solvents can be mixed at a ratio of 1 to 90% by weight, and preferably 1 to 50% by weight per the entire solvent used in the varnish.

Examples of such a solvent include propylene glycol monomethyl ether, ethylene glycol monobutyl ether, diethylene glycol diethyl ether, diethylene glycol dimethyl ether, diethylene glycol monoethyl ether acetate, diethylene glycol monobutyl ether acetate, dipropylene glycol monomethyl ether, propylene glycol monomethyl ether acetate, diethylene glycol monoethyl ether, diacetone alcohol, γ-butyrolactone, ethyl lactate, n-hexyl acetate, and the like, but are not limited thereto. These solvents can be used alone or in combination of two or more.

The viscosity of the varnish of the present invention is appropriately set depending on the thickness and the like of the thin film to be manufactured and the solid content concentration, but usually 1 to 50 mPa·s at 25°C, and the surface tension thereof is usually 20 to 50 mN/m.

Further, the solid content concentration of the charge-transporting varnish is appropriately set in consideration of the viscosity, surface tension, and the like of the varnish, the thickness of the thin film to be manufactured, and the like, but usually about 0.1 to 10.0% by weight, and considering improving applicability of the varnish, the solid content concentration is preferably about 0.5 to 5.0% by weight, and more preferably about 1.0 to 3.0% by weight.

A method of producing the varnish is not particularly limited, but examples thereof include a method of first dissolving the fluorinated alkyl phosphate onium salt in a solvent and sequentially adding the charge-transporting material thereto, and a method of dissolving a mixture of the fluorinated alkyl phosphate onium salt and the charge-transporting material in a solvent.

Further, when a plurality of organic solvents are used, for example, the fluorinated alkyl phosphate onium salt and the charge-transporting material may be first dissolved in a solvent capable of well-dissolving the fluorinated alkyl phosphate onium salt and the charge-transporting material and other solvents may be added thereto, or the fluorinated alkyl phosphate onium salt and the charge-transporting material may be sequentially or simultaneously dissolved in a mixed solvent of a plurality of organic solvents.

In the present invention, from the standpoint of reproducibly obtaining thin films having a high flatness, it is desirable for the charge-transporting varnish to be obtained by dissolving the fluorinated alkyl phosphate onium salt, the charge-transporting compound, and the like in the organic solvent and subsequently filtering the solution using a submicron-order filter or the like.

The charge-transporting thin film of the present invention can be formed on a substrate by applying the above-described charge-transporting varnish on the substrate and baking the applied varnish.

The method of applying the varnish is not particularly limited, but examples thereof include a dip method, a spin coating method, a transfer printing method, a roll coating method, a brush coating method, an inkjet method, a spray method, a slit coating method, and the like, and it is preferred to adjust the viscosity and surface tension of the varnish depending on the application method.

Further, if the varnish of the present invention is used, the baking atmosphere is also not particularly limited, and a thin film having a uniform film formation surface and high charge transportability can be obtained not only in the air atmosphere but also in an inert gas such as nitrogen or in vacuum, but depending on the type of charge-transporting compounds and the like, a thin film having higher charge transportability can be obtained with good reproducibility, by baking the varnish under the air atmosphere.

A baking temperature is appropriately set within a range of about 100 to 260°C, considering the use of the obtained thin film, the charge transportability degree which is imparted to the obtained thin film, a solvent type, a boiling point, and the like, and if the obtained thin film is used as a hole injection layer of an organic EL device, about 140 to 250°C is preferred and about 145 to 240°C is more preferred.

In addition, at the time of baking, for the purpose of expressing a film having higher uniformity or proceeding with a reaction on the substrate, two or more stages of temperature change may be applied and heating may be performed using an appropriate device such as for example, a hot plate or an oven.

The thickness of the charge-transporting thin film is not particularly limited, but is preferably 5 to 200 nm, if the thin film is used as a hole injection layer, a hole transport layer, or a hole injection/transport layer of an organic EL device. As a method of changing the film thickness, there are methods such as changing a solid content concentration in the varnish or changing a solution amount on a substrate at the time of application.

The organic EL device of the present invention has a pair of electrodes and has the charge-transporting thin film of the present invention between the electrodes.

The typical configurations of the organic EL device include the following (a) to (f), but are not limited thereto. In addition, in the following configuration, if necessary, an electron block layer and the like can be provided between the light-emitting layer and a positive electrode, and a hole block layer and the like can be provided between the light-emitting layer and a negative electrode. Further, a hole injection layer, a hole transport layer, or a hole injection/transport layer may have a function as the electron block layer and the like, and an electron injection layer, an electron transport layer, or an electron injection/transport layer may have a function as the hole block layer and the like.
(a) positive electrode/hole injection layer/hole transport layer/light-emitting layer/electron transport layer/electron injection layer/negative electrode
(b) positive electrode/hole injection layer/hole transport layer/light-emitting layer/electron injection/transport layer/negative electrode
(c) positive electrode/hole injection/transport layer/light-emitting layer/electron transport layer/electron injection layer/negative electrode
(d) positive electrode/hole injection/transport layer/light-emitting layer/electron injection/transport layer/negative electrode
(e) positive electrode/hole injection layer/hole transport layer/light-emitting layer/negative electrode
(f) positive electrode/hole injection/transport layer/light-emitting layer/negative electrode

The "hole injection layer", the "hole transport layer", and the "hole injection/transport layer" are layers formed between the light-emitting layer and the positive electrode and have a function of transporting holes from the positive electrode to the light-emitting layer, and if only one layer of hole transporting materials is provided between the light-emitting layer and the positive electrode, it is the "hole injection/transport layer", and if two or more layers of hole transporting materials are provided between the light-emitting layer and the positive electrode, the layer near the positive electrode is the "hole injection layer" and the other layer is the "hole transport layer". In particular, as the hole injection (transport) layer, a thin film which is excellent not only in a hole-accepting property from the positive electrode but also in a hole injecting property to the hole transport (light emitting) layer is used.

The "electron injection layer", the "electron transport layer", and the "electron injection/transport layer" are layers formed between the light-emitting layer and the negative electrode and have a function of transporting electrons from the negative electrode to the light-emitting layer, and if only one layer of hole transporting materials is provided between the light-emitting layer and the negative electrode, it is the "electron injection/transport layer", and if two or more layers of electron transporting materials are provided between the light-emitting layer and the negative electrode, the layer near the negative electrode is the "electron injection layer" and the other layer is the "electron transport layer".

The "light-emitting layer" is an organic layer having a light emitting function, and includes host materials and dopant materials if a doping system is employed. In this case, the host materials promote mainly a recombination of electrons and holes, and has a function of confining excitons in the light-emitting layer, and the dopant materials have a function of efficiently emitting excitons obtained by the recombination. In the case of a phosphorescent element, the host materials mainly have a function of confining excitons produced by a dopant in the light-emitting layer.

The charge-transporting thin film of the present invention can be suitably used as the hole injection layer, the hole transport layer, and the hole injection/transport layer in the organic EL device, and can be more suitably used as the hole injection layer.

Examples of the materials used and the manufacturing method when manufacturing the organic EL device using the charge-transporting varnish of the present invention include the following, but are not limited thereto.

It is preferred that the electrode substrate to be used is cleaned in advance by a liquid cleaning with a detergent, alcohol, pure water, and the like, and for example, it is preferred to perform surface treatment such as a UV ozone treatment and an oxygen-plasma treatment immediately prior to use on the positive electrode substrate. However, if the positive electrode materials include an organic material as a main component, surface treatment may not be performed.

An example of a method of manufacturing the organic EL device having the hole injection layer including the thin film obtained from the charge-transporting varnish of the present invention is as follows.

The charge-transporting varnish of the present invention is applied on a positive electrode substrate and baked by the above-described method and the hole injection layer on the electrode is manufactured.

On the hole injection layer, a hole transport layer, a light-emitting layer, an electron transport layer, an electron injection layer, and a negative electrode are provided in this order. The hole transport layer, the light-emitting layer, the electron transport layer, and the electron injection layer may be formed by either a vapor deposition method or a coating method (wet process), depending on the properties of the materials to be used and the like.

Examples of the positive electrode materials include a transparent electrode represented by indium tin oxide (ITO) or indium zinc oxide (IZO), or a metal positive electrode including a metal represented by aluminum, an alloy thereof, or the like, and it is preferred to perform a flattening treatment. A polythiophene derivative or a polyaniline derivative having high charge transportability can also be used.

In addition, examples of other metals forming the metal positive electrode include scandium, titanium, vanadium, chromium, manganese, iron, cobalt, nickel, copper, zinc, gallium, yttrium, zirconium, niobium, molybdenum, ruthenium, rhodium, palladium, cadmium, indium, scandium, lanthanum, cerium, praseodymium, neodymium, promethium, samarium, europium, gadolinium, terbium, dysprosium, holmium, erbium, thulium, ytterbium, hafnium, thallium, tungsten, rhenium, osmium, iridium, platinum, gold, titanium, lead, bismuth, an alloy thereof, and the like, but are not limited thereto.

Examples of the materials forming the hole transport layer include hole-transporting low molecular weight materials, for example, triarylamines such as a (triphenylamine)dimer derivative, a [(triphenylamine)dimer]spirodimer, N,N'-bis(naphthalen-1-yl)-N,N'-bis(phenyl)-benzidine (α-NPD), N,N'-bis(naphthalen-2-yl)-N,N'-bis(phenyl)-benzidine, N,N'-bis(3-methylphenyl)-N,N'-bis(phenyl)-benzidine, N,N'-bis(3-methylphenyl)-N,N'-bis(phenyl)-9,9-spirobifluorene, N,N'-bis(naphthalene-1-yl)-N,N'-bis(phenyl)-9,9-spirobifluorene, N,N'-bis(3-methylphenyl)-N,N'-bis(phenyl)-9,9-dimethyl-fluorene, N,N'-bis(naphthalen-1-yl)-N,N'-bis(phenyl)-9,9-dimethyl-fluorene, N,N'-bis(3-methylphenyl)-N,N'-bis(phenyl)-9,9-diphenyl-fluorene, N,N'-bis(naphthalen-1-yl)-N,N'-bis(phenyl)-9,9-diphenyl-fluorene, N,N'-bis(naphthalen-1-yl)-N,N'-bis(phenyl)-2,2'-dimethylbenzidine, 2,2',7,7'-tetrakis(N,N-diphenylamino)-9,9-spirobifluorene, 9,9-bis[4-(N,N-bis-biphenyl-4-yl-amino)phenyl]-9H-fluorene, 9,9-bis[4-(N,N-bis-naphthalen-2-yl-amino)phenyl]-9H-fluorene, 9,9-bis[4-(N-naphthalen-1-yl-N-phenylamino)-phenyl]-9H-fluorene, 2,2',7,7'-tetrakis[N-naphthalenyl(phenyl)-amino]-9,9-spirobifluorene, N,N'-bis(phenanthrene-9-yl)-N,N'-bis(phenyl)-benzidine, 2,2'-bis[N,N-bis(biphenyl-4-yl)amino]-9,9-spirobifluorene, 2,2'-bis(N,N-diphenylamino)-9,9-spirobifluorene, di-[4-(N,N-di(p-tolyl)amino)-phenyl]cyclohexane, 2,2',7,7'-tetra(N,N-di(p-tolyl))amino-9,9-spirobifluorene, N,N,N',N'-tetra-naphthalen-2-yl-benzidine, N,N,N',N'-tetra-(3-methylphenyl)-3,3'-dimethylbenzidine, N,N'-di(naphthalenyl)-N,N'-di(naphthalen-2-yl)-benzidine, N,N,N',N'-tetra(naphthalenyl)-benzidine, N,N'-di(naphthalen-2-yl)-N,N'-diphenylbenzidine-1,4-diamine, N¹,N⁴-diphenyl-N¹,N⁴-di(m-tolyl)benzene-1,4-diamine, N²,N²,N⁶,N⁶-tetraphenylnaphthalen-2,6-diamine, tris(4-(quinoline-8-yl)phenyl)amine, 2,2'-bis(3-(N,N-di(p-tolyl)amino)phenyl)biphenyl, 4,4',4"-tris[3-methylphenyl(phenyl)-amino]triphenylamine (m-MTDATA), and 4,4',4"-tris[1-naphthyl(phenyl)amino]-triphenylamine (1-TNATA), oligothiophenes such as 5,5"-bis-{4-[bis(4-methylphenyl)-amino]phenyl}-2,2':5',2"-terthiophene (BMA-3T), and the like.

Examples of the materials forming the light-emitting layer include tris(8-quinolinolat)aluminum(III) (Alq₃), bis(8-quinolinolat)zinc(II) (Znq₂), bis(2-methyl-8-quinolinolat)-4-(p-phenylphenolat)aluminum(III) (BAlq), 4,4'-bis(2,2-diphenylvinyl)biphenyl, 9,10-di(naphthalen-2-yl)anthracene, 2-t-butyl-9,10-di(naphthalen-2-yl)anthracene, 2,7-bis[9,9-di(4-methylphenyl)-fluorene-2-yl]-9,9-di(4-methylphenyl)fluorene, 2-methyl-9,10-bis(naphthalen-2-yl)anthracene, 2-(9,9-spirobifluorene-2-yl)-9,9-spirobifluorene, 2,7-bis(9,9-spirobifluorene-2-yl)-9,9-spirobifluorene, 2-[9,9-di(4-methylphenyl)-fluorene-2-yl]-9,9-di(4-methylphenyl)fluorene, 2,2'-dipyrenyl-9,9-spirobifluorene, 1,3,5-tris(pyrene-1-yl)benzene, 9,9-bis[4-(pyrenyl)-phenyl]-9H-fluorene, 2,2'-bi(9,10-diphenylanthracene), 2,7-dipyrenyl-9,9-spirobifluorene, 1,4-di(pyrene-1-yl)benzene, 1,3-di(pyrene-1-yl)benzene, 6,13-di(biphenyl-4-yl)pentacene, 3,9-di(naphthalen-2-yl)perylene, 3,10-di(naphthalen-2-yl)perylene, tris[4-(pyrenyl)-phenyl]amine, 10,10'-di(biphenyl-4-yl)-9,9'-bianthracene, N,N'-di(naphthalen-1-yl)-N,N'-diphenyl-[1,1':4',1":4",1'''-quarterphenyl]-4,4'''-diamine,4,4'-di[10-(naphthalen-1-yl)anthracen-9-yl]biphenyl, dibenzo{[f,f']-4,4',7,7'-tetraphenyl}diindeno[1,2,3-cd:1',2',3'-lm]perylene, 1-(7-(9,9'-bianthracen-10-yl)-9,9-dimethyl-9H-fluorene-2-yl)pyrene, 1-(7-(9,9'-bianthracen-10-yl)-9,9-dihexyl-9H-fluorene-2-yl)pyrene, 1,3-bis(carbazole-9-yl)benzene, 1,3,5-tris(carbazole-9-yl)benzene, 4,4',4"-tris(carbazole-9-yl)triphenylamine, 4,4'-bis(carbazole-9-yl)biphenyl (CBP), 4,4'-bis(carbazole-9-yl)-2,2'-dimethylbiphenyl, 2,7-bis(carbazole-9-yl)-9,9-dimethylfluorene, 2,2',7,7'-tetrakis(carbazole-9-yl)-9,9-spirobifluorene, 2,7-bis(carbazole-9-yl)-9,9-di(p-tolyl)fluorene, 9,9-bis[4-(carbazole-9-yl)-phenyl]fluorene, 2,7-bis(carbazole-9-yl)-9,9-spirobifluorene, 1,4-bis(triphenylsilyl)benzene, 1,3-bis(triphenylsilyl)benzene, bis(4-N,N-diethylamino-2-methylphenyl)-4-methylphenylmethane, 2,7-bis(carbazole-9-yl)-9,9-dioctylfluorene, 4,4"-di(triphenylsilyl)-p-terphenyl, 4,4'-di(triphenylsilyl)biphenyl, 9-(4-t-butylphenyl)-3,6-bis(triphenylsilyl)-9H-carbazole, 9-(4-t-butylphenyl)-3,6-ditrityl-9H-carbazole, 9-(4-t-butylphenyl)-3,6-bis(9-(4-methoxyphenyl)-9H-fluorene-9-yl)-9H-carbazole, 2,6-bis(3-(9H-carbazole-9-yl)phenyl)-pyridine, triphenyl(4-(9-phenyl-9H-fluorene-9-yl)phenyl)silane, 9,9-dimethyl-N,N-diphenyl-7-(4-(1-phenyl-1H-benzo[d]imidazol-2-yl)phenyl)-9H-fluorene-2-amine, 3,5-bis(3-(9H-carbazole-9-yl)phenyl)pyridine, 9,9-spirobifluorene-2-yl-diphenylphosphine oxide, 9,9'-(5-(triphenylsilyl)-1,3-phenylene)bis(9H-carbazole), 3-(2,7-bis(diphenylphosphoryl)-9-phenyl-9H-fluorene-9-yl)-9-phenyl-9H-carbazole, 4,4,8,8,12,12-hexa(p-tolyl)-4H-8H-12H-12C-azadibenzo[cd, mn]pyrene, 4,7-di(9H-carbazole-9-yl)-1,10-phenanthroline, 2,2'-bis(4-(carbazole-9-yl)phenyl)biphenyl, 2,8-bis(diphenylphosphoryl)dibenzo[b,d]thiophene, bis(2-methylphenyl)diphenylsilane, bis[3,5-di(9H-carbazole-9-yl)phenyl]diphenylsilane, 3,6-bis(carbazole-9-yl)-9-(2-ethylhexyl)-9H-carbazole, 3-(diphenylphosphoryl)-9-(4-(diphenylphosphoryl)phenyl)-9H-carbazole, 3,6-bis[(3,5-diphenyl)phenyl]-9-phenylcarbazole, and the like, and the light-emitting layer may be formed by co-deposition with a light-emitting dopant.

Examples of the light-emitting dopant include 3-(2-benzothiazolyl)-7-(diethylamino)coumarin, 2,3,6,7-tetrahydro-1,1,7,7-tetramethyl-1H,5H,11H-10-(2-benzothiazolyl)quinolizino[9,9a,1gh]coumarin, quinacridone, N,N'-dimethyl-quinacridone, tris(2-phenylpyridine)iridium(III)(Ir(ppy)₃), bis(2-phenylpyridine)(acetylacetonate)iridium(III)(Ir(ppy)₂(acac)), tris[2-(p-tolyl)pyridine]iridium(III)(Ir(mppy)₃), 9,10-bis[N,N-di(p-tolyl)amino]anthracene, 9,10-bis[phenyl(m-tolyl)amino]anthracene, bis[2-(2-hydroxyphenyl)benzothiazolate]zinc(II), N¹⁰,N¹⁰,N^{10'},N^{10'}-tetra(p-tolyl)-9,9'-bianthracen-10,10'-diamine, N¹⁰,N¹⁰,N^{10'},N^{10'}-tetraphenyl-9,9'-bianthracen-10,10'-diamine, N¹⁰,N^{10'}-diphenyl-N¹⁰,N^{10'}-dinaphthalenyl-9,9'-bianthracen-10,10'-diamine, 4,4'-bis(9-ethyl-3-carbazovinylene)-1,1'-biphenyl, perylene, 2,5,8,11-tetra-t-butylperylene, 1,4-bis[2-(3-N-ethylcarbazolyl)vinyl]benzene, 4,4'-bis[4-(di-p-tolylamino)styryl]biphenyl, 4-(di-p-tolylamino)-4'-[(di-p-tolylamino)styryl]stilbene, bis[3,5-difluoro-2-(2-pyridyl)phenyl-(2-carboxypyridyl)]iridium(III), 4,4'-bis[4-(diphenylamino)styryl]biphenyl, bis(2,4-difluorophenylpyridinato)tetrakis(1-pyrazolyl)borate iridium(III), N,N'-bis(naphthalen-2-yl)-N,N'-bis(phenyl)-tris(9,9-dimethylfluorenylene), 2,7-bis{2-[phenyl(m-tolyl)amino]-9,9-dimethyl-fluorene-7-yl}-9,9-dimethyl-fluorene, N-(4-((E)-2-(6((E)-4-(diphenylamino)styryl)naphthalen-2-yl)vinyl)phenyl)-N-phenylbenzeneamine, fac-iridium(III)tris(1-phenyl-3-methylbenzimidazolin-2-ylidene-C,C²'), mer-iridium(III)tris(1-phenyl-3-methylbenzimidazolin-2-ylidene-C,C²'), 2,7-bis[4-(diphenylamino)styryl]-9,9-spirobifluorene, 6-methyl-2-(4-(9-(4-(6-methylbenzo[d]thiazol-2-yl)phenyl)anthracen-10-yl)phenyl)benzo[d]thiazole, 1,4-di[4-(N,N-diphenyl)amino]styrylbenzene, 1,4-bis(4-(9H-carbazole-9-yl)styryl)benzene, (E)-6-(4-(diphenylamino)styryl)-N,N-diphenylnaphthalen-2-amine, bis(2,4-difluorophenylpyridinato)(5-(pyridine-2-yl)-1H-tetrazolate)iridium(III), bis(3-trifluoromethyl-5-(2-pyridyl)pyrazole)((2,4-difluorobenzyl)diphenylphosphinate)-iridium(III), bis(3-trifluoromethyl-5-(2-pyridyl)pyrazolate)(benzyldiphenylphosphinate)-iridium(III), bis(1-(2,4-difluorobenzyl)-3-methylbenzimidazolium)(3-(trifluoromethyl)-5-(2-pyridyl)-1,2,4-triazolate)iridium(III), bis(3-trifluoromethyl-5-(2-pyridyl)pyrazolate)(4',6'-difluorophenylpyridinate)iridium(III), bis(4',6'-difluorophenylpyridinato)(3,5-bis(trifluoromethyl)-2-(2'-pyridyl)pyrolate)iridium(III), bis(4',6'-difluorophenylpyridinato)(3-(trifluoromethyl)-5-(2-pyridyl)-1,2,4-triazolate)iridium(III), (Z)-6-mesityl-N-(6-mesitylquinoline-2(1H)-ylidene)quinoline-2-amine-BF₂, (E)-2-(2-(4-(dimethylamino)styryl)-6-methyl-4H-pyrane-4-ylidene)malononitrile, 4-(dicyanomethylene)-2-methyl-6-julolidine-9-enyl-4H-pyran, 4-(dicyanomethylene)-2-methyl-6-(1,1,7,7-tetramethyljulolidyl-9-enyl)-4H-pyran, 4-(dicyanomethylene)-2-t-butyl-6-(1,1,7,7-tetramethyljulolidine-4-yl-vinyl)-4H-pyran, tris(dibenzoylmethane)phenanthroline europium(III), 5,6,11,12-tetraphenylnaphthacene, bis(2-benzo[b]thiophene-2-yl-pyridine)(acetylacetonate)iridium(III), tris(1-phenylisoquinoline)iridium(III), bis(1-phenylisoquinoline)(acetylacetonate)iridium(III), bis[1-(9,9-dimethyl-9H-fluorene-2-yl)-isoquinoline](acetylacetonate)iridium(III), bis[2-(9,9-dimethyl-9H-fluorene-2-yl)quinoline](acetylacetonate)iridium(III), tris[4,4'-di-t-butyl-(2,2')-bipyridine]ruthenium(III)·bis(hexafluorophosphate), tris(2-phenylquinoline)iridium(III), bis(2-phenylquinoline)(acetylacetonate)iridium(III), 2,8-di-t-butyl-5,11-bis(4-t-butylphenyl)-6,12-diphenyltetracene, bis(2-phenylbenzothiazolate)(acetylacetonate)iridium(III), 5,10,15,20-tetraphenyltetrabenzoporphyrin platinum, osmium(II)bis(3-trifluoromethyl-5-(2-pyridine)-pyrazolate)dimethylphenylphosphine, osmium(II)bis(3-(trifluoromethyl)-5-(4-t-butylpyridyl)-1,2,4-triazolate)diphenylmethylphosphine, osmium(II)bis(3-(trifluoromethyl)-5-(2-pyridyl)-1,2,4-triazole)dimethylphenylphosphine, osmium(II)bis(3-(trifluoromethyl)-5-(4-t-butylpyridyl)-1,2,4-triazolat)dimethylphenylphosphine, bis[2-(4-n-hexylphenyl)quinoline](acetylacetonate)iridium(III), tris[2-(4-n-hexylphenyl)quinoline]iridium(III), tris[2-phenyl-4-methylquinoline]iridium(III), bis(2-phenylquinoline)(2-(3-methylphenyl)pyridinate)iridium(III), bis(2-(9,9-diethyl-fluorene-2-yl)-1-phenyl-1H-benzo[d]imidazolat)(acetylacetonate)iridium(III), bis(2-phenylpyridine)(3-(pyridine-2-yl)-2H-chromen-2-onate)iridium(III), bis(2-phenylquinoline)(2,2,6,6-tetramethylheptane-3,5-dionate)iridium(III), bis(phenylisoquinoline)(2,2,6,6-tetramethylheptane-3,5-dionate)iridium(III), iridium(III)bis(4-phenylthieno[3,2-c]pyridinato-N,C^{2'})acetylacetonate, (E)-2-(2-t-butyl-6-(2-(2,6,6-trimethyl-2,4,5,6-tetrahydro-1H-pyrrolo[3,2,1-ij]quinoline-8-yl)vinyl)-4H-pyran-4-ylidene)malononitrile, bis(3-trifluoromethyl-5-(1-isoquinolyl)pyrazolate)(methyldiphenylphosphine)ruthenium, bis[(4-n-hexylphenyl)isoquinoline](acetylacetonate)iridium(III), platinum(II)octaethylporphine, bis(2-methyldibenzo[f,h]quinoxaline)(acetylacetonate)iridium(III), tris[(4-n-hexylphenyl)quinoquinoline]iridium(III), and the like.

Examples of the materials forming the electron transport layer include 8-hydroxyquinolinolate-lithium, 2,2',2"-(1,3,5-benzinetolyl)-tris(1-phenyl-1-H-benzimidazole), 2-(4-biphenyl)5-(4-t-butylphenyl)-1,3,4-oxadiazole, 2,9-dimethyl-4,7-diphenyl-1,10-phenanthroline, 4,7-diphenyl-1,10-phenanthroline, bis(2-methyl-8-quinolinolat)-4-(phenylphenolat)aluminum, 1,3-bis[2-(2,2'-bipyridine-6-yl)-1,3,4-oxadiazol-5-yl]benzene, 6,6'-bis[5-(biphenyl-4-yl)-1,3,4-oxadiazol-2-yl]-2,2'-bipyridine, 3-(4-biphenyl)-4-phenyl-5-t-butylphenyl-1,2,4-triazole, 4-(naphthalen-1-yl)-3,5-diphenyl-4H-1,2,4-triazole, 2,9-bis(naphthalen-2-yl)-4,7-diphenyl-1,10-phenanthroline, 2,7-bis[2-(2,2'-bipyridine-6-yl)-1,3,4-oxadiazol-5-yl]-9,9-dimethylfluorene, 1,3-bis[2-(4-t-butylphenyl)-1,3,4-oxadiazol-5-yl]benzene, tris(2,4,6-trimethyl-3-(pyridine-3-yl)phenyl)boran, 1-methyl-2-(4-(naphthalen-2-yl)phenyl)-1H-imidazo[4,5f][1,10]phenanthroline, 2-(naphthalen-2-yl)-4,7-diphenyl-1,10-phenanthroline, phenyl-dipyrenylphosphineoxide, 3,3',5,5' -tetra[(m-pyridyl)-phen-3-yl]biphenyl, 1,3,5-tris[(3-pyridyl)-phen-3-yl]benzene, 4,4'-bis(4,6-diphenyl-1,3,5-triazin-2-yl)biphenyl, 1,3-bis[3,5-di(pyridine-3-yl)phenyl]benzene, bis(10-hydroxybenzo[h]quinolinato)beryllium, diphenylbis(4-(pyridine-3-yl)phenyl)silane, 3,5-di(pyrene-1-yl)pyridine, and the like.

Examples of the materials forming the electron injection layer includes lithium oxide (Li₂O), magnesium oxide (MgO), alumina (Al₂O₃), lithium fluoride (LiF), sodium fluoride (NaF), magnesium fluoride (MgF₂), cesium fluoride (CsF), strontium fluoride (SrF₂), molybdenum trioxide (MoO₃), aluminum, Li(acac), lithium acetate, lithium benzoate, and the like.

Examples of the negative electrode materials include aluminum, a magnesium-silver alloy, an aluminum-lithium alloy, lithium, sodium, potassium, cesium, and the like.

Further, other examples of the method of manufacturing the organic EL device having a hole injection layer including the thin film obtained from the charge-transporting varnish of the present invention are as follows.

In the manufacture of the EL device, instead of performing a vacuum deposition operation of a hole transport layer, a light-emitting layer, an electron transport layer, and an electron injection layer, an organic EL device having a charge-transporting thin film formed by the charge-transporting varnish of the present invention can be manufactured, by sequentially forming the hole transport layer (hereinafter, referred to as a hole-transporting polymer layer) and the light-emitting layer (hereinafter, referred to as a light-emitting polymer layer).

Specifically, the charge-transporting varnish of the present invention is applied on a positive electrode substrate to manufacture a hole injection layer by the above-described method, a hole-transporting polymer layer and a light-emitting polymer layer are sequentially formed thereon, and a negative electrode is further deposited to form an organic EL device.

As the positive electrode and negative electrode materials to be used, those as described above are used, and the same cleaning treatment and surface treatment can be performed.

Examples of the method of forming the hole-transporting polymer layer and the light-emitting polymer layer include a method in which a solvent is added to hole-transporting polymer materials or light-emitting polymer materials, or materials to which a dopant material is added, which are then dissolved or uniformly dispersed therein, and applied on a hole injection layer or a hole transporting polymer layer and each baked, thereby forming a thin film.

Examples of the hole-transporting polymer materials include poly[(9,9-dihexylfluorenyl-2,7-diyl)-co-(N,N'-bis{p-butylphenyl}-1,4-diaminophenylene)], poly[(9,9-dioctylfluorenyl-2,7-diyl)-co-(N,N'-bis{p-butylphenyl}-1,1'-biphenylene-4,4-diamine)], poly[(9,9-bis {1'-penten-5'-yl}fluorenyl-2,7-diyl)-co-(N,N'-bis{p-butylphenyl}-1,4-diaminophenylene)], poly[N,N'-bis(4-butylphenyl)-N,N'-bis(phenyl)-benzidine]-end capped with polysilsesquinoxane, poly[(9,9-didioctylfluorenyl-2,7-diyl)-co-(4,4'-(N-(p-butylphenyl))diphenylamine)], and the like.

Examples of the light-emitting polymer layer materials include polyfluorene derivatives such as poly(9,9-dialkylfluorene) (PDAF), polyphenylenevinylene derivatives such as poly(2-methoxy-5-(2'-ethylhexoxy)-1,4-phenylenevinylene) (MEH-PPV), polythiophene derivatives such as poly(3-alkylthiophene) (PAT), polyvinylcarbazole (PVCz), and the like.

Examples of the solvent include toluene, xylene, chloroform, and the like, and examples of a dissolution or uniform dispersion method include methods such as stirring, heating and stirring, and ultrasonic dispersion.

The application method is not particularly limited, but examples thereof include an inkjet method, a spray method, a dip method, a spin coating method, a transfer printing method, a roll coating method, a brush coating method, and the like. In addition, it is preferred that the application is performed under an inert gas such as nitrogen or argon.

Examples of the baking method include a method of heating in an oven or a hot plate under an inert gas or under a vacuum.

An example of a method of manufacturing the EL device having the hole transport layer including the thin film obtained from the charge-transporting varnish of the present invention is as follows.

A hole injection layer is formed on a positive electrode substrate. On the layer, the charge-transporting varnish of the present invention is applied and baked by the above-described method, thereby manufacturing a hole transport layer.

On the hole transport layer, a light-emitting layer, an electron transport layer, an electron injection layer, and a negative electrode are provided in this order. A method of forming the light-emitting layer, the electron transport layer, and the electron injection layer, and specific examples thereof are as described above. Further, the hole injection layer may be formed by either a vapor deposition method or a coating method (wet process), depending on the properties of the materials to be used and the like.

Examples of the materials forming the hole injection layer include copper phthalocyanine, titanium oxide phthalocyanine, platinum phthalocyanine, pyrazino[2,3-f][1,10]phenanthroline-2,3-dicarbonitrile, N,N,N',N'-tetrakis(4-methoxyphenyl)benzidine, 2,7-bis[N,N-bis(4-methoxyphenyl)amino]-9,9-spirobifluorene, 2,2'-bis[N,N-bis(4-methoxy-phenyl)amino]-9,9-spirobifluorene, N,N'-diphenyl-N,N'-di[4-(N,N-ditolylamino)phenyl]benzidine, N,N'-diphenyl-N,N'-di[4-(N,N-diphenylamino)phenyl]benzidine, N⁴,N^{4'}-(biphenyl-4,4'-diyl)bis(N⁴,N^{4'},N^{4'}-triphenylbiphenyl-4,4'-diamine)N¹,N^{1'}-(biphenyl-4,4'-diyl)bis(N¹-phenyl-N⁴,N^{4'}-di-m-tolylbenzene-1,4-diamine), charge-transporting materials described in WO 2004/043117, WO 2004/105446, WO 2005/000832, WO 2005/043962, WO 2005/042621, WO 2005/107335, WO 2006/006459, WO 2006/025342, WO 2006/137473, WO 2007/049631, WO 2007/099808, WO 2008/010474, WO 2008/032617, WO 2008/032616, WO 2008/129947, WO 2009/096352, WO 2010/041701, WO 2010/058777, WO 2010/058776, WO 2013/042623, WO 2013/129249, WO 2014/115865, WO 2014/132917, WO 2014/141998, and WO 2014/132834, and the like.

Examples of the positive electrode materials, the materials forming the light-emitting layer, the light-emitting dopant, the electron transport layer, and the electron block layer, and the negative electrode materials include those as described above.

An example of a method of manufacturing the organic EL device having a hole injection/transport layer including the thin film obtained from the charge-transporting varnish of the present invention is as follows.

On a positive electrode substrate, a hole injection/transport layer is formed, and on the hole injection/transport layer, a light-emitting layer, an electron transport layer, an electron injection layer, and a negative electrode are provided in this order. A method of forming the light-emitting layer, the electron transport layer, and the electron injection layer, and specific examples thereof are as described above.

Examples of the positive electrode materials, the materials forming the light-emitting layer, the light-emitting dopant, the electron transport layer, and the electron block layer, and the negative electrode materials include those as described above.

In addition, if necessary, a hole block layer, an electron block layer, and the like may be provided between the electrodes and each of the layers described above. Examples of the materials forming the electron block layer include tris(phenylpyrazole)iridium, and the like.

The materials constituting the positive electrode, the negative electrode, and the layer formed between the positive electrode and the negative electrode are different depending on whether the device is provided with a bottom emission structure or a top emission structure, and thus, appropriate materials are selected considering such a point.

Usually, in the device having the bottom emission structure, a transparent positive electrode is used on a substrate side and light is extracted from the substrate side, while in the device having the top emission structure, a reflective positive electrode composed of metal is used and light is extracted from the transparent electrode (negative electrode) in the opposite direction to the substrate, and thus, for example, in the case of the positive electrode materials, the transparent positive electrode such as ITO is used in manufacture of the device having the bottom emission structure and the reflective positive electrode such as Al/Nd is used in manufacture of the device having the top emission structure.

The organic EL device of the present invention may be sealed with a water-collecting agent and the like, if necessary, according to a standard method, for preventing deterioration of the characteristics.

### EXAMPLES

Hereinafter, the present invention is described in detail, with reference to the Examples and the Comparative Examples, but the present invention is not limited to the following Examples. In addition, apparatuses to be used are as follows.
(1) ¹H, ¹⁹F-NMR:
   a nuclear magnetic resonance apparatus AL-300 manufactured by JEOL Ltd.
(2) Substrate cleaning:
   a substrate cleaning apparatus (low-pressure plasma system) manufactured by Choshu Industry Co., Ltd.
(3) Application of varnish:
   Spincoater MS-A100 manufactured by Mikasa Co., Ltd.
(4) Film thickness measurement:
   a fine shape measurement device Surf coder ET-4000 manufactured by Kosaka Laboratory, Ltd.
(5) Surface observation of film:
   a confocal laser microscope real time scanning laser microscope 1LM21D manufactured by Lasertec Corporation
(6) Manufacture of EL device:
   a multifunctional deposition device system C-E2L1G1-N manufactured by Choshu Industry Co., Ltd.
(7) Measurement of brightness and the like of EL device:
   a multi-channel IVL measurement device manufactured by EHC Co., Ltd.
(8) Lifetime measurement (brightness half-life measurement) of EL device:
   an organic EL brightness lifetime evaluation system PEL-105S manufactured by EHC Co., Ltd.

### [1] Synthesis of Fluorinated Alkyl Phosphate Onium Salt

### [Synthesis Example 1] Synthesis of (4-phenylthiophenyl)diphenylsulfonium tris(pentafluoroethyl)trifluorophosphate salt (P-1)

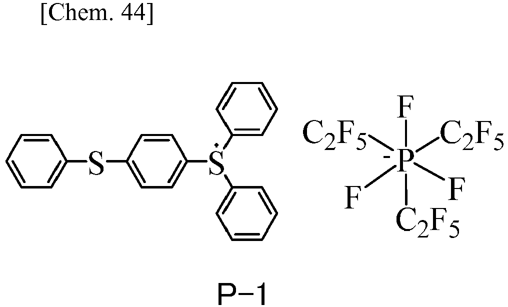

P-1 represented by the above formula was synthesized according to the method described in Production Example 9 of JP 5828679.

### [2] Preparation of Charge-Transporting Varnish

### [Working Example 1-1]

A charge-transporting varnish was obtained by adding 5 g of 3-phenoxytoluene and 5 g of 4-methoxytoluene to a mixture of 343 mg of T-1 represented by the following formula synthesized according to the method described in Production Example 12 of WO 2015/050253 and 183 mg of P-1 obtained in Synthesis Example 1, stirring and dissolving the mixture at room temperature to obtain a solution, and filtering the solution with a syringe filter having a pore size of 0.2 µm.

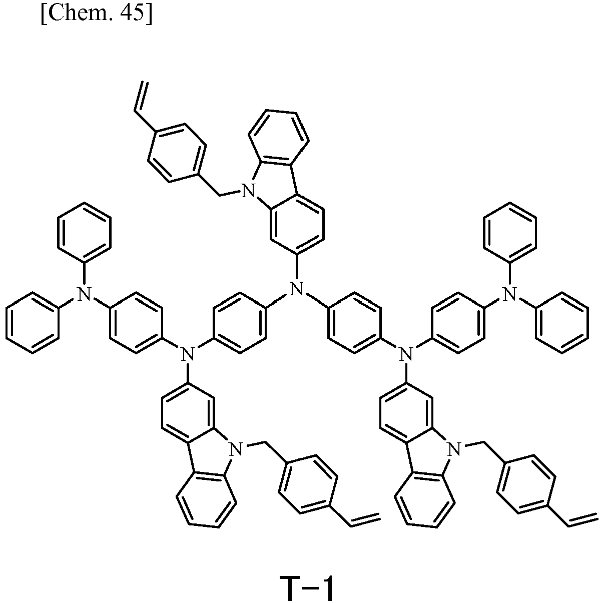

### [Working Example 1-2]

A charge-transporting varnish was obtained by adding 4 g of 3-phenoxytoluene and 4 g of 4-methoxytoluene to a mixture of 291 mg of T-2 represented by the following formula synthesized according to the method described in Production Example 23 of WO 2015/050253 and 130 mg of P-1 obtained in Synthesis Example 1, stirring and dissolving the mixture at room temperature to obtain a solution, and filtering the solution with a syringe filter having a pore size of 0.2 µm.

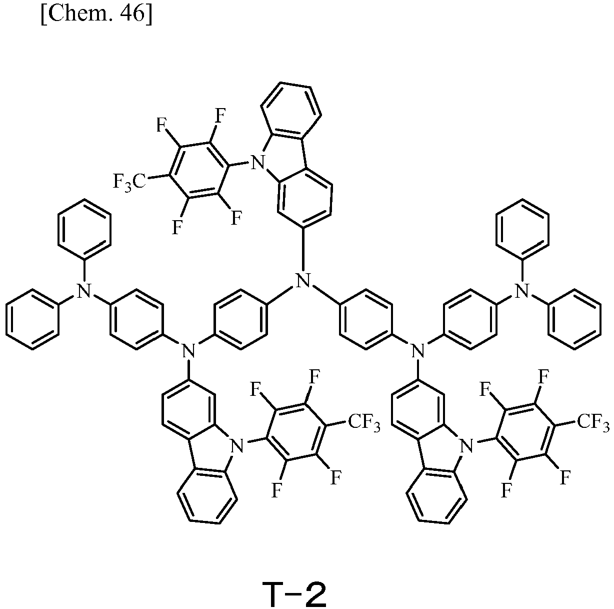

### [Working Example 1-3]

A charge-transporting varnish was obtained by adding 4 g of 3-phenoxytoluene and 4 g of 4-methoxytoluene to a mixture of 129 mg of the above T-1, 154 mg of the above T-2, and 138 mg of P-1 synthesized in Synthesis Example 1, stirring and dissolving the mixture at room temperature to obtain a solution, and filtering the solution with a syringe filter having a pore size of 0.2 µm.

### [3] Production of Organic EL Device and Evaluation of Device Characteristics

### [Working Example 2-1]

The varnish obtained in Working Example 1-1 was applied onto an ITO substrate using a spin coater, and then baked at 200°C for 10 minutes under the atmosphere, thereby forming a uniform 50 nm thin film on the ITO substrate. A glass substrate with dimensions of 25 mm × 25 mm × 0.7 t having indium-tin oxide (ITO) patterned on the surface to a film thickness of 150 nm was used as the ITO substrate, and impurities on the surface were removed from the substrate with an O₂ plasma cleaning system (150 W, 30 seconds), before using the substrate.

Next, a vapor deposition system (degree of vacuum, 1.0×10⁻⁵ Pa) was used to form a 30 nm film of α-NPD (N,N'-di(1-naphthyl)-N,N'-diphenylbenzidine) at a rate of 0.2 nm/s onto the ITO substrate on which the thin film was formed. Then, CBP and Ir(PPy)₃ were co-deposited. Co-deposition was carried out to a 40 nm lamination while controlling the rate of deposition such that the concentration of Ir(PPy)₃ becomes 6%. Then, thin films of BAlq, lithium fluoride, and aluminum were then successively laminated, thereby giving an organic EL device. At this time, vapor deposition was carried out under the conditions of a rate of 0.2 nm/s for BAlq and aluminum, and a rate of 0.02 nm/s for lithium fluoride. The film thicknesses were set to, respectively, 20 nm, 0.5 nm, and 80 nm.

In addition, in order to prevent the characteristic degradation by the influence of oxygen in the air, water, and the like, after the organic EL device was sealed with a sealing substrate, the characteristics were evaluated. Sealing was performed in the following order. The organic EL device was stored between the sealing substrates in the nitrogen atmosphere at an oxygen concentration of 2 ppm or less and a dew point of -85°C or less, and the sealing substrate was bonded together by an adhesive (manufactured by MORESCO Corporation Mores Moisture Cut WB90US(P)). At this time, a water-collecting agent (manufactured by Dynic Corporation, HD-071010W-40) was stored in a sealing substrate with the organic EL device. After the bonded sealing substrate was irradiated with UV light (wavelength: 365 nm, irradiation amount: 6,000 mJ/cm²), the sealing substrate was subjected to annealing treatment at 80°C for 1 hour to cure the adhesive.

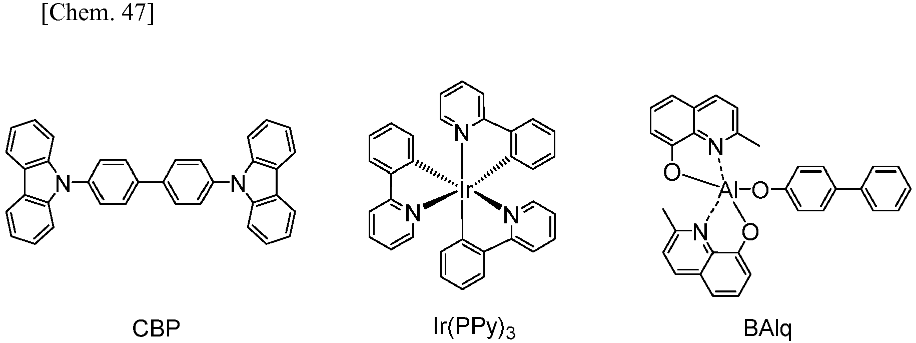

### [Working Example 2-2]

An organic EL device was produced in the same manner as in Working Example 2-1, except that the charge-transporting varnish obtained in Working Example 1-2 was used instead of the charge-transporting varnish obtained in Working Example 1-1.

### [Working Example 2-3]

An organic EL device was produced in the same manner as in Working Example 2-1, except that the charge-transporting varnish obtained in Working Example 1-3 was used instead of the charge-transporting varnish obtained in Working Example 1-1.

The characteristics of the devices of Examples 2-1 to 2-3 were evaluated. A driving voltage, a current density, current efficiency, luminous efficiency, and external luminescence quantum efficiency (EQE) when the device emitted light at 5,000 cd/m² are shown in Table 19. A half-life of brightness of the device (initial brightness of 5,000 cd/m²) is shown in Table 20.

**[Table 19]**

| | Driving voltage (V) | Current density (mA/cm²) | Current efficiency (cd/A) | Emission efficiency (lm/W) | EQE (%) |
|---|---|---|---|---|---|
| Working Example 2-1 | 9.4 | 28.5 | 17.6 | 5.9 | 5.1 |
| Working Example 2-2 | 9.4 | 28.3 | 17.6 | 5.9 | 5.1 |
| Working Example 2-3 | 9.6 | 28.7 | 17.4 | 5.7 | 5.1 |

**[Table 20]**

| | Half-life (hours) |
|---|---|
| Working Example 2-1 | 166 |
| Working Example 2-2 | 258 |
| Working Example 2-3 | 258 |

As shown in Tables 19 and 20, the EL device provided with the charge-transporting thin film of the present invention was suitably driven. Further, the life characteristics were also excellent.

## Claims

1. A charge-transporting varnish comprising: a charge-transporting material, a fluorinated alkyl phosphate onium salt, and an organic solvent, wherein
the fluorinated alkyl phosphate onium salt includes a fluorinated alkyl phosphate onium salt consisting of an anion of formula (a1) and a countercation: 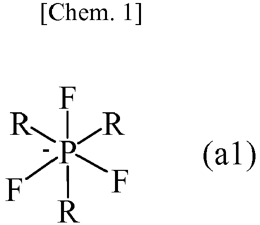 wherein R is an alkyl group of 1 to 10 carbon atoms, a fluoroalkyl group of 1 to 10 carbon atoms, an aralkyl group of 7 to 10 carbon atoms, or a fluoroaralkyl group of 7 to 10 carbon atoms.

2. The charge-transporting varnish of claim 1, wherein the charge-transporting material is at least one selected from an aniline derivative and a thiophene derivative.

3. The charge-transporting varnish of claim 2, wherein the charge-transporting material is the aniline derivative.

4. A charge-transporting thin film, obtained from the charge-transporting varnish of any one of claims 1 to 3.

5. An organic electroluminescent device, comprising the charge-transporting thin film of claim 4.

6. A method of producing a charge-transporting thin film, the method comprising applying the charge-transporting varnish of any one of claims 1 to 3 on a substrate, and evaporating a solvent.
